# EUROPÄISCHE PATENTSCHRIFT

(11) **EP 4 327 929 B1**
(45) Veröffentlichungstag und Bekanntmachung des Hinweises auf die Patenterteilung: **21.05.2025**
(21) Anmeldenummer: 22192285.9
(22) Anmeldetag: 26.08.2022
(51) Int. Cl.: B01J 8/02, B01J 8/04, B01J 19/24, C07C 29/152

(54) **METHANOLSYNTHESE-PLATTENREAKTOR**
METHANOL SYNTHESIS PLATE REACTOR
RÉACTEUR À PLAQUES POUR LA SYNTHÈSE DU MÉTHANOL

(43) Veröffentlichungstag der Anmeldung: 28.02.2024
(73) Patentinhaber: L'AIR LIQUIDE, SOCIETE ANONYME POUR L'ETUDE ET L'EXPLOITATION DES PROCEDES GEORGES CLAUDE, 75007 Paris (FR)
(72) Erfinder: STROZYK, Michael, 60439 Frankfurt am Main (DE); OELMANN, Tobias, 60439 Frankfurt am Main (DE)
(74) Vertreter: Air Liquide

(56) Entgegenhaltungen:
- JP-A- S60 168 530
- US-A- 4 743 432
- US-A- 5 874 051
- US-A1- 2002 048 541

## Beschreibung

### Technisches Gebiet der Erfindung

Die Erfindung betrifft einen Methanolsynthese-Reaktor, insbesondere einen Methanolsynthese-Reaktor umfassend zumindest zwei integrierte Prozesseinheiten.

### Stand der Technik

Bei nicht integrierten Lösungen zur Herstellung von kondensierbaren Reaktionsprodukten, beispielsweise Rohmethanol, finden die einzelnen Prozessschritte in separaten Einzelpositionen oder Einzelapparaten statt. Für das Beispiel der Methanolsynthese bestehen diese Prozessschritte zumindest aus der Reaktion von Synthesegas zu Methanol, der Kühlung des Reaktionsgemischs zur Kondensation von Rohmethanol, und der Trennung (Abscheidung) des kondensierten Rohmethanols vom nicht reagierten Synthese-gas (Restgas) und inerten Bestandteilen des Gasgemischs.

Bei der Verwendung von Einzelapparaten werden Prozessmedien der Prozesseinheit durch Stutzen in einen Druckapparat und von dort aus in die jeweiligen innenliegenden Apparatebauteile eingeleitet, dort verteilt beziehungsweise gesammelt und anschließend ausgeleitet. Dies geschieht durch den jeweiligen Apparat betreffende dedizierte Apparate-Öffnungen und -Einbauten zum einen, und zum anderen durch verbindende Elemente wie Rohrleitungen und Armaturen, und je nach der relativen Lage der Prozesseinheiten über weitere Fördereinrichtungen wie Pumpen.

Um mehrere Prozesseinheiten zu kombinieren, sind somit mehrere Druckapparate und externe Rohrleitungen erforderlich, welche separat gefertigt und auf Fundamenten oder in einem Stahlbau innerhalb einer Anlage installiert werden müssen. Dies führt zu hohen wirtschaftlichen Aufwendungen bezüglich der erforderlichen Investitionen (CAPEX) und auch der anfallenden Betriebskosten (OPEX). Dies ist insbesondere begründet durch die Vielzahl der erforderlichen Druckmäntel und Stutzen, sowie Rohrleitungen und Fördereinrichtungen. Diese Faktoren führen zu hohen Druckverlusten und Wärmeverlusten über das damit einhergehende große Gesamtvolumen und die damit einhergehende große Gesamtoberfläche, sowie zu einem höheren Energieeinsatz für die erforderlichen Fördereinrichtungen.

JP S60 168530 A offenbart einen Methanolsynthese-Reaktor mit mehreren mit einem Katalysator gefüllten Reaktionsrohren, welche in mehrere obere und untere Stufen unterteilt sind. Das zu reagierende Gas wird durch die Reaktionsrohre in Reihe geleitet, während die Außenfläche jedes Reaktionsrohres zur Kühlung mit Wasser in Kontakt gebracht wird. Dabei wird die Temperatur des Wassers, das mit der Außenfläche eines stromabwärts angeordneten Reaktionsrohrs in Berührung kommt, auf einer niedrigeren Temperatur gehalten als das Wasser, das mit der Außenfläche eines stromaufwärts angeordneten Reaktionsrohrs in Berührung kommt.

US 4 743 432 A offenbart einen vertikal angeordneten Reaktor zur Erzeugung von Methanol. Dieser weist ein zylindrisches Gehäuse mit das Katalysatorbett im Inneren des Gehäuses durchsetzenden Austauscherrohren auf, sowie einen das Katalysatorbett tragenden, gasdurchlässigen Boden oder Netz im unteren Reaktorabschnitt. Unterhalb des gasdurchlässigen Bodens oder Netzes des zweituntersten und des nächsthöheren Katalysatorbetts sind in Höhe der Zu- oder Abströmöffnungen für das Gas horizontale gasundurchlässige Trennwände angeordnet.

US 2002/048541 A1 offenbart einen Reaktor zur Durchführung einer stark exothermen katalytischen Reaktion in einem Prozessfluid, ausgerüstet mit in einem Abstand parallel zueinander angeordneter Platten, die in miteinander gegenüberliegenden seitlichen Begrenzungsflächen flache Kanäle bilden, wobei ein Teil der Kanäle einen festen Katalysator enthält und das Prozessfluid leitet und ein anderer Teil der Kanäle ein Wärmeübertragungsmedium in indirektem Wärmekontakt mit dem Prozessfluid führt. Die Platten sind eben oder mit Rillen oder Rippen versehen und die Platten auf der dem Prozessfluid zugewandten Oberfläche sind mindestens teilweise mit dem Katalysator beschichtet.

US 5 874 051 A offenbart ein Verfahren und eine Vorrichtung zur selektiven katalytischen Oxidation von Kohlenmonoxid. Dabei wird ein Gasgemisch und ein zusätzlich zugegebenes oxidierendes Gas durch einen Reaktor geleitet, der den Katalysator enthält. Das oxidierende Gas wird an mehreren Stellen entlang des Mischgasstroms mit einem gesteuerten oder geregelten Durchflussvolumen zugegeben. Der Mischgasstrom wird durch statische Mischstrukturen im Einlassbereich des Oxidationsreaktors passiv gekühlt.

### Beschreibung der Erfindung

Eine Aufgabe der vorliegenden Erfindung besteht darin, die vorgenannten Nachteile des Standes der Technik zumindest teilweise zu überwinden.

Eine weitere Aufgabe der vorliegenden Erfindung besteht darin einen Reaktor zur Synthese von Methanol bereitzustellen, welcher es ermöglicht die Zahl verbindender Rohrleitungen, einzelner Druckmäntel und Apparateböden zu reduzieren.

Eine weitere Aufgabe der vorliegenden Erfindung besteht darin einen Reaktor zur Syntheses von Methanol bereitzustellen, welcher zumindest zwei Prozessfunktionen erfüllt und dabei ein möglichst geringes Gesamtvolumen und eine möglichst geringe Gesamtoberfläche aufweist.

Eine weitere Aufgabe der vorliegenden Erfindung besteht darin einen Reaktor zur Synthese von Methanol bereitzustellen, welcher in Bezug auf die Erfüllung von zumindest zwei Prozessfunktionen eine möglichst geringe Gesamtfertigungszeit erfordert.

Eine weitere Aufgabe der vorliegenden Erfindung besteht darin einen Reaktor zur Synthese von Methanol bereitzustellen, welcher möglichst keine oder wenige Fördereinrichtungen in Bezug auf die zu erfüllenden Prozessfunktionen erfordert.

Eine weitere Aufgabe der vorliegenden Erfindung besteht darin einen Reaktor zur Synthese von Methanol bereitzustellen, welcher so konstruiert ist, dass Materialspannungen in den metallbasierten Bauteilen minimiert werden.

Ein Beitrag zur mindestens teilweisen Erfüllung mindestens einer der obigen Aufgaben wird durch die unabhängigen Ansprüche geleistet. Die abhängigen Ansprüche stellen bevorzugte Ausführungsformen bereit, die zur mindestens teilweisen Erfüllung mindestens einer der Aufgaben beitragen. Bevorzugte Ausgestaltungen von Bestandteilen einer erfindungsgemäßen Kategorie sind, soweit zutreffend, ebenso bevorzugt für gleichnamige oder entsprechende Bestandteile einer jeweils anderen erfindungsgemäßen Kategorie.

Die Ausdrücke "aufweisend", "umfassend" oder "beinhaltend" etc. schließen nicht aus, dass weitere Elemente, Inhaltsstoffe etc. enthalten sein können. Der unbestimmte Artikel "ein" schließt nicht aus, dass eine Mehrzahl vorhanden sein kann.

Gemäß einem ersten Aspekt der Erfindung wird ein Methanolsynthese-Reaktor zur Herstellung von Methanol aus einem Synthesegasgemisch vorgeschlagen, aufweisend
(a) einen Druckmantel mit einem Innenraum, wobei in dem Innenraum zumindest zwei fluidisch miteinander verbundene Prozesseinheiten übereinander angeordnet sind;
(b) eine erste Prozesseinheit, wobei die erste Prozesseinheit als Reaktorstufe RS1 zur Synthese von Methanol aus einem Synthesegas-Strom an einem Katalysatorbett CB1 konfiguriert ist, wodurch ein methanolhaltiger Produktstrom RP1 erzeugbar ist, und
   die erste Prozesseinheit als Plattenwärmeübertrager ausgestaltet ist, wobei die erste Prozesseinheit dabei eine Vielzahl von vertikal und parallel zueinander angeordneten Platten mit durchströmbaren Platteninnenräumen aufweist, und zwischen benachbarten Platten Plattenzwischenräume ausgebildet sind, wobei
   in den Plattenzwischenräumen ein fester Methanolsynthese-Katalysator angeordnet ist, welcher das Katalysatorbett CB1 bildet, und die erste Prozesseinheit so konfiguriert ist, dass die Plattenzwischenräume von dem Synthesegas-Strom und von dem methanolhaltigen Produktstrom RP1 von oben nach unten durchströmbar sind und die Platteninnenräume von einem ersten Kühlmedien-Strom CM1 von unten nach oben durchströmbar sind, wodurch
   der methanolhaltige Produktstrom RP1 durch den ersten Kühlmedien-Strom CM1 im Gegenstrom kühlbar ist, und wobei
   die erste Prozesseinheit Mittel zum Abziehen des methanolhaltigen Produktstroms RP1 aus der ersten Prozesseinheit aufweist;
(c) eine unterhalb der ersten Prozesseinheit angeordnete zweite Prozesseinheit, wobei
   die zweite Prozesseinheit als Plattenwärmeübertrager ausgestaltet ist, wobei die zweite Prozesseinheit dabei eine Vielzahl von vertikal und parallel zueinander angeordneten Platten mit durchströmbaren Platteninnenräumen aufweist, und zwischen benachbarten Platten Plattenzwischenräume ausgebildet sind, wobei
   die zweite Prozesseinheit so konfiguriert ist, dass die Platteninnenräume von einem zweiten Kühlmedien-Strom CM2 von unten nach oben durchströmbar sind, und wobei
      - (c1) die zweite Prozesseinheit als Kühlstufe ausgestaltet ist und dabei so konfiguriert ist, dass die Plattenzwischenräume von dem methanolhaltigen Produktstrom RP1 von oben nach unten durchströmbar sind, wodurch der methanolhaltige Produktstrom RP1 durch den zweiten Kühlmedien-Strom CM2 im Gegenstrom kühlbar ist, oder
      - (c2) die zweite Prozesseinheit als Reaktorstufe RS2 ausgestaltet ist und dabei zur Synthese von Methanol aus dem aus der ersten Prozesseinheit abziehbaren methanolhaltigen Produktstrom RP1 an einem Katalysatorbett CB2 konfiguriert ist, wodurch ein methanolhaltiger Produktstrom RP2 erzeugbar ist, und wobei in den Plattenzwischenräumen ein fester Methanolsynthese-Katalysator angeordnet ist, welcher das Katalysatorbett CB2 bildet, und die zweite Prozesseinheit so konfiguriert ist, dass die Plattenzwischenräume von dem methanolhaltigen Produktstrom RP1 und dem methanolhaltigen Produktstrom RP2 von oben nach unten durchströmbar sind, wodurch der methanolhaltige Produktstrom RP2 durch den zweiten Kühlmedien-Strom CM2 im Gegenstrom kühlbar ist.

Unter einer "Vielzahl" von Platten wird erfindungsgemäß eine Mehrzahl von Platten verstanden, vorzugsweise jedoch mehr als zwei Platten, insbesondere mindestens 5 Platten, oder mindestens 10 Platten, oder mindestens 25 Platten, oder mindestens 50 Platten.

Die zumindest zwei Prozesseinheiten im Innenraum des Druckmantels beinhalten die erste und die zweite Prozesseinheit.

Erfindungsgemäß werden zumindest zwei Prozesseinheiten in einen gemeinsamen Druckmantel integriert. Unter einem Druckmantel wird dabei eine umhüllende Struktur verstanden, welche höheren Innendrücken standhält, insbesondere Innendrücken deutlich oberhalb des Atmosphärendrucks, beispielsweise Drücken von mehr als 5 bar.

Die erste Prozesseinheit erfüllt die Funktion einer Reaktorstufe zur Synthese von Methanol aus einem in die Reaktorstufe einleitbaren Synthesegas-Strom, hier als Reaktorstufe RS1 bezeichnet. Zur Erfüllung dieser Funktion ist die erste Prozesseinheit als Plattenwärmeübertrager ausgestaltet. Mit anderen Worten, die erste Prozesseinheit weist eine Struktur eines Plattenwärmeübertragers auf.

Dabei sind zwei benachbarte Platten des Plattenwärmeübertragers so voneinander beabstandet, dass sich zwischen zwei benachbarten Platten ein Plattenzwischenraum ausbildet.

In den Plattenzwischenräumen der ersten Prozesseinheit ist ein fester Methanolsynthese-Katalysator angeordnet. Dabei bildet die Gesamtheit des in den Plattenzwischenräumen der ersten Prozesseinheit angeordneten Methanolsynthese-Katalysators das Katalysatorbett, hier als Katalysatorbett CB1 bezeichnet.

Bei dem Methanolsynthese-Katalysator handelt es sich um einen dem Fachmann bekannten Methanolsynthese-Katalysator, beispielsweise auf Kupfer-Basis. Der Methanolsynthese-Katalysator kann dabei beispielsweise als Schüttung oder strukturierte Packung ausgestaltet sein.

Die Methanolsynthese-Katalysator aufweisenden Plattenzwischenräume sind so konfiguriert, dass sie mit dem Synthesegas-Strom und dem Produktstrom RP1 von oben nach unten durchströmbar sind.

Alternativ formuliert weist die erste Prozesseinheit Mittel auf welche so konfiguriert sind, dass die Plattenzwischenräume mit dem Synthesegas-Strom und dem Produktstrom RP1 von oben nach unten durchströmbar sind.

Dadurch findet eine exotherme Reaktion des Synthesegases des Synthesegas-Stroms am Methanolsynthese-Katalysator des Katalysatorbetts CB1 statt. Dabei bildet sich der Produktstrom RP1. Entsprechend sind die Plattenzwischenräume auch mit dem Produktstrom RP1 von oben nach unten durchströmbar.

Dabei wird insbesondere der methanolhaltige Produkstrom RP1 durch den die Platteninnenräume von unten nach oben durchströmenden ersten Kühlmedien-Strom CM1 im Gegenstrom gekühlt.

Das Synthesegas des Synthesegas-Stroms weist ein Kohlenstoffoxid, also Kohlenmonoxid oder Kohlendioxid, oder eine Mischung daraus auf. Ferner weist das Synthesegas des Synthesegas-Stroms Wasserstoff auf. Das Synthesegas kann darüber hinaus generell inerte oder unter den Bedingungen der Methanolsynthesese inerte Bestandteile aufweisen. Beispiele sind Stickstoff oder Methan. Der Produktstrom RP1 weist zumindest Methanol und Wasser als kondensierbare Bestandteile sowie gegebenenfalls nicht erwünschte kondensierbare und nicht kondensierbare Nebenprodukte auf. Ferner weist der Produktstrom RP1 gegebenenfalls nicht umgesetztes Synthesegas auf.

Die Platten der ersten Prozesseinheit weisen durchströmbare Innenräume auf. Dabei sind die Innenräume der Platten von einem ersten Kühlmedien-Strom CM1 durchströmbar. Dabei ist die erste Prozesseinheit so konfiguriert, dass die Platteninnenräume von dem ersten Kühlmedien-Strom CM1 von unten nach oben durchströmbar sind.

Alternativ formuliert weist die erste Prozesseinheit Mittel auf welche so konfiguriert sind, dass die Platteninnenräume von dem Kühlmedien-Strom CM1 von unten nach oben durchströmbar sind.

Die Platteninnenräume der ersten Prozesseinheit sind dabei vorzugsweise in vertikaler Richtung durchströmbar, insbesondere in vertikaler Richtung von unten nach oben von dem ersten Kühlmedien-Strom durchströmbar.

Die Plattenzwischenräume der ersten Prozesseinheit sind dabei vorzugsweise in vertikaler Richtung durchströmbar, insbesondere in vertikaler Richtung von oben nach unten von dem Synthesegasstrom und dem Produktstrom RP1 durchströmbar.

Die zweite Prozesseinheit erfüllt entweder die Funktion einer Kühlstufe oder die Funktion einer zweiten Reaktorstufe RS2.

Aus der ersten Prozesseinheit, welche als Reaktorstufe RS1 konfiguriert ist, ist der methanolhaltige Produktstrom RP1 abziehbar und in die zweite Prozesseinheit einleitbar. In der zweiten Prozesseinheit wird dieser Produktstrom RP1 entweder gekühlt (Alternative c1) oder weiter zu einem methanolhaltigen Produktstrom RP2 umgesetzt (Alternative c2). In letzterem Fall wird im methanolhaltigen Produktstrom RP1 nicht umgesetztes Synthesegas, also verbliebenes Synthesegas oder Restgas, zu Methanol umgesetzt.

Zur Erfüllung dieser alternativen Funktionen ist auch die zweite Prozesseinheit als Plattenwärmeübertrager ausgestaltet. Mit anderen Worten, die zweite Prozesseinheit weist eine Struktur eines Plattenwärmeübertragers auf.

Dabei sind zwei benachbarte Platten des Plattenwärmeübertragers so voneinander beabstandet, dass sich zwischen zwei benachbarten Platten ein Plattenzwischenraum ausbildet.

Die Platten weisen durchströmbare Innenräume auf, welche von einem zweiten Kühlmedien-Strom CM2 durchströmt werden. Dabei ist die zweite Prozesseinheit so konfiguriert, dass die Platteninnenräume von dem zweiten Kühlmedienstrom CM2 von unten nach oben durchströmbar sind.

Alternativ formuliert weist die erste Prozesseinheit Mittel auf welche so konfiguriert sind, dass die Platteninnenräume von dem zweiten Kühlmedien-Strom CM2 von unten nach oben durchströmbar sind.

Die Platteninnenräume der zweiten Prozesseinheit sind dabei vorzugsweise in vertikaler Richtung von dem zweiten Kühlmedien-Strom CM2 durchströmbar, insbesondere in vertikaler Richtung von unten nach oben von dem zweiten Kühlmedien-Strom durchströmbar.

Entsprechend Alternative c1 ist die zweite Prozesseinheit als Kühlstufe ausgestaltet. Entsprechend dieser Konfiguration sind die Plattenzwischenräume der zweiten Prozesseinheit von dem methanolhaltigen Produktstrom RP1 von oben nach unten durchströmbar. Alternativ formuliert weist die zweite Prozesseinheit entsprechend Alternative c1 Mittel auf welche so konfiguriert sind, dass die Plattenzwischenräume von dem methanolhaltigen Produktstrom RP1 von oben nach unten durchströmbar sind. Die Plattenzwischenräume sind dabei vorzugsweise in vertikaler Richtung von dem methanolhaltigen Produktstrom RP1 durchströmbar.

Dabei wird der methanolhaltige Produkstrom RP1 durch den die Platteninnenräume von unten nach oben durchströmenden zweiten Kühlmedien-Strom CM2 gekühlt. Insbesondere ist die zweite Prozesseinheit gemäß Alternative c1 dabei so konfiguriert, dass der methanolhaltige Produktstrom RP1 so weit gekühlt wird, dass eine Kondensation von Methanol und Wasser aus dem methanolhaltigen Produktstrom RP1 erfolgt.

Entsprechend Alternative c2 ist die zweite Prozesseinheit als Reaktorstufe RS2 ausgestaltet.

Entsprechend dieser Konfiguration c2 ist in den Plattenzwischenräumen der zweiten Prozesseinheit ein fester Methanolsynthese-Katalysator angeordnet. Dabei bildet die Gesamtheit des in den Plattenzwischenräumen der zweiten Prozesseinheit angeordneten Methanolsynthese-Katalysators das Katalysatorbett, hier als Katalysatorbett CB2 bezeichnet.

Bei dem Methanolsynthese-Katalysator handelt es sich um einen dem Fachmann bekannten Methanolsynthese-Katalysator, beispielsweise auf Kupfer-Basis. Der Methanolsynthese-Katalysator kann dabei beispielsweise als Schüttung oder strukturierte Packung ausgestaltet sein.

Die Methanolsynthese-Katalysator aufweisenden Plattenzwischenräume sind so konfiguriert, dass sie mit dem Produktstrom RP1 und dem Produktstrom RP2 von oben nach unten durchströmbar sind. Alternativ formuliert weist die die zweite Prozesseinheit Mittel auf welche so konfiguriert sind, dass die Plattenzwischenräume mit dem Produktstrom RP1 und dem Produktstrom RP2 von oben nach unten durchströmbar sind. Dadurch findet eine exotherme Reaktion des Produktstroms RP1 am Methanolsynthese-Katalysator des Katalysatorbettes CB2 statt. Dabei bildet sich der Produktstrom RP2. Entsprechend sind die Plattenzwischenräume auch mit dem Produktstrom RP2 von oben nach unten durchströmbar. Die zweite Prozesseinheit bildet in diesem Fall eine zweite Reaktorstufe RS2. In dieser zweiten Reaktorstufe RS2 wird im Produktstrom RP1 enthaltenes Synthesegas zu Methanol umgesetzt, welches in der ersten Reaktorstufe RS1 aufgrund der Ausbildung des für die Methanolsynthese typischen thermodynamischen Gleichgewichts nicht zu Methanol umgesetzt werden konnte. Dieses verbleibende Synthesegas oder Restgas bildet also einen Teil des Produktstroms RP1.

Der Produktstrom RP2 weist zumindest Methanol und Wasser als kondensierbare Bestandteile sowie gegebenenfalls nicht erwünschte kondensierbare und nicht kondensierbare Nebenprodukte auf. Ferner weist der Produktstrom RP2 gegebenenfalls nach wie vor nicht umgesetztes Synthesegas auf.

Entsprechend der Konfiguration gemäß Alternative c2 sind die Plattenzwischenräume der zweiten Prozesseinheit von dem methanolhaltigen Produktstrom RP1 und methanolhaltigen Produktstrom RP2 von oben nach unten durchströmbar. Alternativ formuliert weist die zweite Prozesseinheit entsprechend Alternative c2 Mittel auf welche so konfiguriert sind, dass die Plattenzwischenräume von dem methanolhaltigen Produktstrom RP1 und dem methanolhaltigen Produktstrom RP2 von oben nach unten durchströmbar sind. Die Plattenzwischenräume sind dabei vorzugsweise in vertikaler Richtung von dem methanolhaltigen Produktstrom RP1 und dem methanolhaltigen Produktstrom RP2 durchströmbar.

Dabei wird der methanolhaltige Produkstrom RP2 durch den die Platteninnenräume von unten nach oben durchströmenden zweiten Kühlmedien-Strom CM2 im Gegenstrom gekühlt.

Die erste und zweite Prozesseinheit sind innerhalb des Druckmantels übereinander angeordnet, wobei die zweite Prozesseinheit unterhalb der ersten Prozesseinheit angeordnet ist. Beide Prozesseinheiten sind fluidisch miteinander verbunden. Insbesondere sind die erste und zweite Prozesseinheit bezüglich des Synthesegas-Stroms sowie der methanolhaltigen Produktströme RP1 und RP2 fluidisch miteinander verbunden, insbesondere auf der Seite der Plattenzwischenräume fluidisch miteinander verbunden.

Vorzugsweise sind die erste und zweite Prozesseinheit fluchtend hintereinander, das heißt entlang einer bezüglich der ersten und zweiten Prozesseinheit gemeinsamen Geraden oder Achse, angeordnet. Vorzugsweise ist die zweite Prozesseinheit also gegenüber der ersten Prozesseinheit nicht versetzt angeordnet und umgekehrt.

Der Methanolsynthese-Reaktor ist gemäß einer Ausführungsform als einstufiger Reaktor mit einer Reaktorstufe RS1 oben und einer Kühlstufe unten ausgestaltet. Gemäß einer weiteren Ausführungsform ist der Methanolsynthese-Reaktor als zweistufiger Reaktor mit einer ersten Reaktorstufe RS1 oben und einer zweiten Reaktorstufe RS2 unten ausgestaltet.

Der Methanolsynthese-Reaktor kann darüber hinaus weitere Prozesseinheiten beinhalten. Beispielsweise kann der Methanolsynthese-Reaktor gemäß Alternative c1 unterhalb der zweiten Prozesseinheit eine dritte Prozesseinheit aufweisen, welche als Kondensator ausgestaltet ist. Gemäß einem weiteren Beispiel kann der Methanolsynthese-Reaktor gemäß Alternative c1 unterhalb der dritten Prozesseinheit eine vierte Prozesseinheit aufweisen, welche als Gas-Flüssigkeit-Separator ausgestaltet ist. Gemäß einem weiteren Beispiel kann der Methanolsynthese-Reaktor gemäß Alternative c2 unterhalb der zweiten Prozesseinheit eine dritte Prozesseinheit aufweisen, welche als Kühlstufe ausgestaltet ist. Gemäß einem weiteren Beispiel kann der Methanolsynthese-Reaktor gemäß Alternative c2 unterhalb der dritten Prozesseinheit eine vierte Prozesseinheit aufweisen, welche als Kondensator ausgestaltet ist. Gemäß einem weiteren Beispiel kann der Methanolsynthese-Reaktor gemäß Alternative c2 unterhalb der vierten Prozesseinheit eine fünfte Prozesseinheit aufweisen, welche als Gas-Flüssigkeit-Separator ausgestaltet ist. Auch sind Ausführungsformen denkbar, in denen die Kühlstufe bereits als Kondensator ausgestaltet ist, so dass in der Kühlstufe bereits eine vollständige Kondensation kondensierbarer Produkte und Nebenprodukte erfolgt.

Bei dem Kühlmedium für den ersten Kühlmedienstrom CM1 und/oder zweiten Kühlmedienstrom CM2 kann es sich um jedes beliebige geeignete gasförmige oder flüssige Kühlmedium handeln.

Vorzugsweise handelt es sich bei dem Kühlmedium für die erste Prozesseinheit um siedendes Wasser, insbesondere siedendes Kesselspeisewasser. Eine bevorzugte Ausführungsform des Reaktors ist daher dadurch gekennzeichnet, dass der erste Kühlmedien-Strom CM1 siedendes Kesselspeisewasser umfasst. Insbesondere ist der erste Kühlmedien-Strom CM1 beim Durchströmen der Platteninnenräume der ersten Prozesseinheit verdampfbar.

Die erste Prozesseinheit ist in diesem Fall als wassergekühlte Reaktorstufe RS1 ausgestaltet, erfüllt also die Funktion einer wassergekühlten Reaktorstufe. Das Kühlmedium verdampft insbesondere durch die bei der Bildung des methanolhaltigen Produktstroms RP1 aufgenommene thermische Energie. Die zur Verdampfung erforderliche thermische Energie entspricht dabei der Verdampfungsenthalpie des Kühlmediums.

Vorzugsweise handelt es sich beim Kühlmedium für die zweite Prozesseinheit um ein gasförmiges Kühlmedium. Die zweite Prozesseinheit ist gemäß dieser Ausführungsform entweder als gasgekühlte Kühlstufe entsprechend Alternative c1 oder als gasgekühlte Reaktorstufe RS2 entsprechend Alternative c2 ausgestaltet. In letzterem Fall erfüllt die zweite Prozesseinheit die Funktion einer gasgekühlten Reaktorstufe.

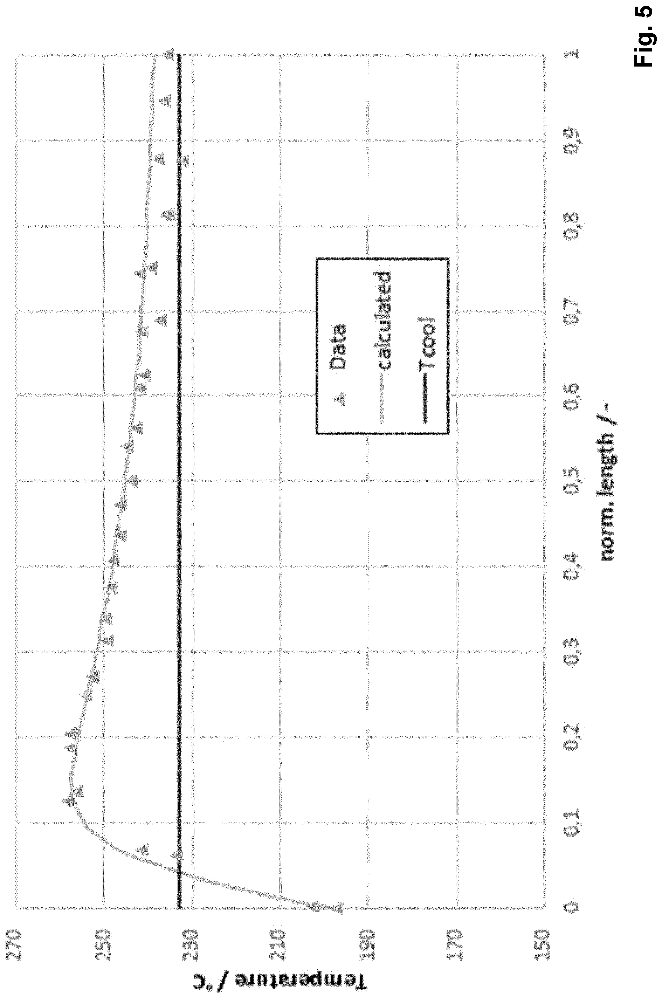

Figur 5 zeigt ein messtechnisch und durch eine Simulation ermitteltes typisches Temperaturprofil eines Katalysatorbetts über die Länge eines Methanol-Reaktors. Dieses Temperaturprofil kann räumlich vom Eintritt in den Reaktor in Richtung Austritt in vier Teile unterteilt werden, wie nachsehend erläutert.

Ein erster Teil des Katalysatorbetts dient der Erwärmung des Synthesegases, wobei Wärme vom Kühlmittel auf das Synthesegas und den Katalysator übertragen wird. In Zuge dessen beginnt allmählich die Reaktion zur Methanolbildung, bei der aufgrund des exothermen Charakters der Reaktion Wärme erzeugt und die Temperatur sowohl des Katalysators als auch des Gasgemisches (Synthesegas und gasförmiges Methanol/Wasser, sowie nicht-reagiertes Synthesegas) erhöht wird. Im weiteren Verlauf der Reaktion entspricht die Temperatur des Katalysatorbetts und des Gasgemischs in etwa der Temperatur des Kühlmittels.

In einem zweiten Teil des Katalysatorbetts schreitet die Reaktion weiter fort, wobei weiter Wärme erzeugt und das Katalysatorbett und das Gasgemisch weiter erwärmt werden. Die Geschwindigkeit der Wärmeerzeugung in diesem zweiten Teil des Katalysatorbettes ist schneller als die Wärmeübertragung vom Kühlmittel, so dass die Temperaturen des Gasgemischs und des Katalysatorbetts über die Temperatur des Kühlmittels steigen. Dabei erwärmt die bei der Reaktion erzeugte Wärme zunächst den festen Katalysator. Anschließend erfolgt Wärmeübertragung vom Katalysator auf das Prozessgasgemisch, um den Katalysator zu kühlen. Anschließend überträgt das Prozessgasgemisch die Wärme auf das im Reaktor verwendete Kühlmittel. Eine weitere Art der Wärmeübertragung ist Wärmekonvektion vom festen Katalysator auf die Reaktoreinbauten. Die Temperatur steigt in diesem Teil des Katalysatorbetts weiter über die des Kühlmittels. Im Verlauf der Reaktion werden die Edukte des Prozessgasgemischs (Kohlenoxide und Wasserstoff) weiter verbraucht und mehr und mehr Rohmethanol als Teil des Prozessgasgemischs wird erzeugt. Da es sich bei der katalytischen Methanolsynthese um eine Gleichgewichtsreaktion handelt, nähert sich die Reaktionsgeschwindigkeit und damit auch die Wärmeproduktionsrate bei Erreichen der Gleichgewichtskonzentration von Edukten und Produkten einem Grenzwert.

In einem dritten Teil des Katalysatorbetts verlangsamt sich die Geschwindigkeit der Wärmeproduktion, da sich die Reaktion den Gleichgewichtsbedingungen nähert. Die Wärmeübertragung vom Katalysator auf das Prozessgasgemisch und letztlich auf das Kühlsystem setzt sich dennoch fort und ermöglicht es, die Temperatur des Katalysatorbetts wieder zu senken.

In einem letzten, vierten Teil des Katalysatorbetts befindet sich die Reaktion im Gleichgewicht, ohne dass eine nennenswerte Wärmeproduktion stattfindet. In diesem Teil des Katalysatorbetts sinkt die Temperatur weiter in Richtung der Kühlmitteltemperatur.

Bezüglich der ersten als auch der zweiten Prozesseinheit gilt, dass die Platten vertikal angeordnet sind und die reagierenden oder zu kühlenden Prozessgase innerhalb der Plattenzwischenräume von oben nach unten geführt werden. Gemäß des oben näher erläuterten Temperaturprofils wird die Temperatur auf der Plattenzwischenraumseite von oben nach unten somit im weitesten Sinne fallen, es bildet sich also über den längsten Bereich der Prozesseinheit ein Temperaturgradient mit fallender Temperatur von oben nach unten aus. Dies gilt prinzipiell noch eindeutiger, wenn die Plattenzwischenräume der zweiten Prozesseinheit wie im Falle der Alternative c1 nicht mit Katalysator gefüllt sind. In diesem Fall fällt die Temperatur des Produktstroms RP1 in der zweiten Prozesseinheit mit fortschreitender Kühlung von oben nach unten über die gesamte Länge der Prozesseinheit kontinuierlich ab.

Da die Dichte eines Mediums bei höheren Temperaturen relativ gesehen niedriger ist als die Dichte des gleichen Mediums bei niedrigeren Temperaturen, bildet sich im Betrieb des Reaktors auch ein Dichtegradient mit einer niedrigen Dichte in einer oberen Zone des Reaktors oder einer Prozesseinheit hin zu einer höheren Dichte in einer unteren Zone des Reaktors oder einer Prozesseinheit aus. Die beabsichtigte Strömungsrichtung des jeweiligen Prozessgasgemischs auf der Plattenzwischenraumseite von oben nach unten wird dadurch unterstützt, da sich schwerere Komponenten (Komponenten mit höherer Dichte) durch die Wirkung der Gravitation in einem unteren Bereich des Reaktors oder einer Prozesseinheit sammeln. Schließlich wird die beabsichtigte Strömungsrichtung des jeweiligen Gemischs weiterhin auch durch die vertikale Anordnung der Platten der Prozesseinheiten begünstigt.

Die Vorteile des erfindungsgemäßen Reaktors erstrecken sich weiterhin auf die Führung der jeweiligen Kühlmedien. Erfindungsgemäß werden die Kühlmedien auf der Platteninnenraumseite der durchströmbaren Platten von unten nach oben geführt, während das jeweilig zu kühlende Prozessgasgemisch auf der Plattenzwischenraumseite von oben nach unten geführt wird. Das Kühlmedium tritt also in einem unteren Bereich in die jeweilige Prozesseinheit ein, und tritt in einem oberen Bereich aus dieser aus. Dabei wärmt es sich in Strömungsrichtung von unten nach oben kontinuierlich durch die Abkühlung des Prozessgasgemisches auf und/oder geht kontinuierlich von einer flüssigen Phase in eine gasförmige Phase über. Der vorgenannte Temperaturgradient und/oder Dichtegradient bildet sich somit nicht nur auf der Platteninnenraumseite, sondern auch auf der Plattenzwischenraumseite aus.

Vereinfacht lässt sich daraus ableiten, dass die jeweilige Prozesseinheit in ihrem Kopfbereich am heißesten ist und im Sumpfbereich am kältesten. Weiterhin weisen die Medien der jeweiligen Prozesseinheit im Kopfbereich im Wesentlichen die niedrigste Dichte auf und im Sumpfbereich die höchste Dichte. Für die Alternative c1 gilt dies im Prinzip für den gesamten Reaktor, da die zweite Prozesseinheit lediglich der Kühlung dient. Je nach Auslegung der Kühlstufe gemäß Alternative c2 neigt das sich auf der Plattenzwischenraumseite befindende Gemisch außerdem mehr oder weniger stark zur Kondensation.

Durch die Kombination der vorgenannten Effekte ist es in Bezug auf den erfindungsgemäßen Reaktor möglich, sowohl bezüglich der Prozessgasgemische auf der Plattenzwischenraumseite (Synthesegas-Strom und Produktströme RP1 und RP2) als auch bezüglich der Kühlmedien auf der Platteninnenraumseite, vollständig auf Fördereinrichtungen mit erzwungener Strömung, wie beispielsweise Pumpen, zu verzichten.

Handelt es sich bei dem Kühlmedium der ersten Prozesseinheit um siedendes Wasser, insbesondere siedendes Kesselspeisewasser, dann entspricht die zur Verdampfung erforderliche thermische Energie der Verdampfungsenthalpie des Kühlmediums. Da die Vorrichtung aufgrund der vorgenannten Effekte in einer oberen Zone im Betrieb heiß ist, wird die Verdampfung in der oberen Zone begünstigt. Dabei bildet sich, wie oben erläutert, auf der Platteninnenraumseite der ersten Prozesseinheit ein Dichtegradient mit einer niedrigen Dichte in der oberen Zone und hoher Dichte in der unteren Zone aus. Dies führt zum sogenannten Thermosyphon-Effekt, welcher einen Naturumlauf des Kühlmediums ermöglicht, ohne dass wie oben beschrieben eine Fördereinrichtung mit erzwungener Strömung erforderlich wäre.

In vorteilhafter Weise, durch die Ausbildung vorgenannter Temperatur- und Dichtegradienten, wird also das sich erhitzende Kühlmedium innerhalb der Platteninnenräume der ersten und/oder zweiten Prozesseinheit nach oben "gesaugt", während das sich abkühlende und/oder kondensierende Prozessgasgemisch auf der Seite der Plattenzwischenräume der ersten und/oder zweiten Prozesseinheit nach unten "fällt".

Der sich durch die erfindungsgemäße Anordnung der ersten und zweiten Prozesseinheit und die erfindungsgemäße Strömungsführung im Betrieb ausbildende Temperaturgradient findet sich nicht nur in den strömenden Medien, sondern entsprechend auch in den metallischen Strukturen der Vorrichtung wieder, da sich die Wärme der Medien entsprechend auf die metallischen Strukturen überträgt. Da der Temperaturgradient kontinuierlich fallend von einem oberen Bereich zu einem unteren Bereich auch in den metallischen Strukturen der Vorrichtung verläuft und im Wesentlichen keine Temperaturspitzen aufweist, werden mögliche thermische Spannungen aufgrund lokal auftretender großer Temperaturunterschiede auf ein Minimum reduziert. Die erste und zweite Prozesseinheit sowie der Druckmantel sind insbesondere aus einem Metall oder einer Metall-Legierung gefertigt. Vorzugsweise sind zumindest die medienführenden Komponenten der ersten und zweiten Prozesseinheit und des Druckmantels aus einem Metall oder einer Metall-Legierung gefertigt.

Eine Ausführungsform des erfindungsgemäßen Reaktors ist dadurch gekennzeichnet, dass die Platten der ersten Prozesseinheit und/oder die Platten der zweiten Prozesseinheit als Kissenplatten ausgestaltet sind.

Kissenplatten anstatt konventioneller Wärmeübertrager-Platten mit gerader Form bieten insbesondere bezüglich der erfindungsgemäßen Vorrichtung Vorteile in Bezug auf mechanische Stabilität und der Effizienz der Wärmeübertragung. Eine Verbesserung der Effizienz der Wärmeübertragung gegenüber konventionellen Platten ergibt sich insbesondere in Bezug auf die Erzeugung von Dampf auf den Platteninnenseiten bei Erreichen des Nukleationspunktes und der nachfolgenden daraus resultierenden Dampfblasenbildung. Kissenplatten-Wärmeübertrager sind beispielsweise näher in der DE 10 2016 005 999 A1 beschrieben.

Eine Ausführungsform des erfindungsgemäßen Reaktors ist dadurch gekennzeichnet, dass die zweite Prozesseinheit so konfiguriert ist, dass die Platteninnenräume von dem Synthesegas-Strom durchströmbar sind, wobei der Synthesegas-Strom die Funktion des zweiten Kühlmedien-Stroms CM2 erfüllt, wodurch ein vorgewärmter Synthesegas-Strom erzeugbar ist.

Um den Einsatz von Kühlmittel zu minimieren, wird der für die Methanolsynthese vorgesehene Synthesegas-Strom in vorteilhafter Weise als Kühlmittel CM2 in der zweiten Prozesseinheit verwendet. In diesem Fall erfüllt die zweite Prozesseinheit entweder die Funktion einer gasgekühlten Kühlstufe (Alternative c1) oder die Funktion einer gasgekühlten Reaktorstufe (Alternative c2). Dabei wird der Synthesegas-Strom in vorteilhafter Weise vorgewärmt, wodurch die Wärmeintegration des erfindungsgemäßen Reaktors verbessert wird. Nach Einleiten des vorgewärmten Synthesegas-Stroms auf die Plattenzwischenraumseite der ersten Prozesseinheit zur Erzeugung des methanolhaltigen Produktstroms RP1 wird die für die Umsetzung am Katalysator erforderliche Temperatur schneller erreicht. Zusätzlich kann auf eine externe Vorrichtung zur Vorwärmung des Synthesegas-Stroms, beispielsweise auf einen Vorwärmer, verzichtet werden.

Eine Ausführungsform des erfindungsgemäßen Reaktors ist daher dadurch gekennzeichnet, dass der Methanolsynthese-Reaktor Mittel zum Ausleiten des vorgewärmten Synthesegas-Stroms aus der zweiten Prozesseinheit aufweist, sowie Mittel zum Einleiten des vorgewärmten Synthesegasstroms in die erste Prozesseinheit zum Erzeugen des methanolhaltigen Produktstroms RP1 aufweist.

Eine Ausführungsform des erfindungsgemäßen Reaktors ist dadurch gekennzeichnet, dass die Platteninnenräume der ersten Prozesseinheit und die Platteninnenräume der zweiten Prozesseinheit zumindest teilweise fluchtend hintereinander angeordnet sind und/oder die Plattenzwischenräume der ersten Prozesseinheit und die Plattenzwischenräume der zweiten Prozesseinheit zumindest teilweise fluchtend hintereinander angeordnet sind.

Unter einer fluchtenden Anordnung hintereinander ist dabei zu verstehen, dass zwei in Flussrichtung der Prozessmedien (Synthesegas-Strom, methanolhaltiger Produktstrom RP1, methanolhaltiger Produktstrom RP2) hintereinander angeordnete Platten der ersten und zweiten Prozesseinheit in Bezug auf die Längsachsen ihrer Platteninnenräume oder Plattenzwischenräume entlang dieser Längsachsen angeordnet sind.

Insbesondere erfolgt diese Anordnung, ohne dass die jeweilige Platte der ersten Prozesseinheit in Bezug auf die betreffende Längsachse versetzt zur Platte der zweiten Prozesseinheit angeordnet ist.

Weiter bevorzugt sind die beiden hintereinander angeordneten Platten der ersten und zweiten Prozesseinheit dabei so angeordnet, dass diese bei gleichen Querschnittsflächen in Bezug auf diese Querschnittsflächen kongruent angeordnet sind, das heißt dass ihre beiden Querschnittsflächen deckungsgleich sind, wenn diese entlang der Längsachse verschoben und zur Deckung gebracht würden.

Vorzugsweise ist der Reaktor dabei so ausgestaltet, dass die fluchtende Anordnung für alle Platten der ersten Prozesseinheit im Verhältnis zu allen Platten der zweiten Prozesseinheit gilt. Das heißt jede Platte der ersten Prozesseinheit hat eine fluchtend hintereinander zu ihr angeordnete Platte als Gegenstück in der zweiten Prozesseinheit.

Durch die Maßnahme der fluchtenden Anordnung kann das Volumen des erfindungsgemäßen Reaktors minimiert werden. Mit anderen Worten, das Verhältnis des beanspruchten Volumens zur zur Verfügung gestellten nutzbaren Fläche wird minimiert. Unter der nutzbaren Fläche wird dabei diejenige Fläche verstanden, welche zur Kühlung der Prozessmedien durch die Kühlmedien oder zur Synthese von Methanol aus den Prozessmedien genutzt werden kann.

Eine Ausführungsform des erfindungsgemäßen Reaktors ist dadurch gekennzeichnet, dass der Methanolsynthese-Reaktor eine Stützstruktur aufweist, wobei die Stützstruktur als zumindest teilweise unterhalb einer Prozesseinheit angeordnete Abstützung und/oder als zumindest teilweise oberhalb einer Prozesseinheit angeordnete Aufhängung ausgestaltet ist und dabei eine mechanische Verbindung zwischen einer Prozesseinheit und dem Druckmantel ausbildet.

Eine "Stützstruktur" im Sinne der Erfindung kann alternativ auch als "Stützelement" bezeichnet werden.

Der erfindungsgemäße Reaktor weist vorzugsweise zumindest eine Stützstruktur auf. Die Stützstruktur bildet eine mechanische Verbindung zwischen einer Prozesseinheit und dem Druckmantel aus. Die mechanische Verbindung stellt einen Fixpunkt zwischen einer Prozesseinheit und dem Druckmantel dar.

Je nach Konfiguration kann für den gesamten Reaktor eine einzige Stützstruktur ausreichend sein, welche eine mechanische Verbindung zwischen einer Prozesseinheit und dem Druckmantel ausbildet. Es können auch mehrere solche Stützstrukturen erforderlich sein, beispielsweise eine Stützstruktur je Prozesseinheit. Bei der mechanischen Verbindung zwischen der jeweiligen Prozesseinheit und dem Druckmantel kann es sich um eine kraftschlüssige Verbindung und/oder eine stoffschlüssige Verbindung handeln.

Ein Beispiel für eine kraftschlüssige Verbindung ist eine Schraubverbindung. Ein Beispiel für eine stoffschlüssige Verbindung ist eine Schweißverbindung.

Die Stützstruktur ist als Aufhängung oder als Abstützung ausgestaltet. Im Falle einer Aufhängung ist die Stützstruktur zumindest teilweise, also teilweise oder vollständig oberhalb der jeweiligen Prozesseinheit angeordnet. Im Falle einer Abstützung ist die Stützstruktur zumindest teilweise, also teilweise oder vollständig unterhalb der jeweiligen Prozesseinheit angeordnet. Gemäß einer Ausführungsform ist die Abstützung vollständig unterhalb einer Prozesseinheit angeordnet und die Aufhängung vollständig oberhalb einer Prozesseinheit angeordnet.

Es ist von Vorteil, die jeweiligen Stützstrukturen nicht vollständig neben einer jeweiligen Prozesseinheit, sondern zumindest teilweise oberhalb oder unterhalb einer Prozesseinheit anzuordnen und mit der jeweiligen Prozesseinheit und dem Druckmantel mechanisch zu verbinden. Dadurch und insbesondere bei einer Anordnung vollständig oberhalb oder unterhalb einer Prozesseinheit können die Stützstrukturen oder Verbindungselemente an einer Stelle positioniert werden, an welcher sie den Innendurchmesser des Reaktors nicht signifikant beeinflussen. Die parallele Anbringung, das heißt Anbringung auf gleicher Höhe der Platten der Prozesseinheit zwischen Prozesseinheit und Wandung des Mantels würde den Querschnitt des Reaktors merklich vergrößern, insbesondere bezüglich des Durchmessers des Druckmantels. Der Druckmantel müsste gemäß Kesselformel entsprechend dickwandiger ausgeführt werden, was von Nachteil wäre.

Weiterhin ermöglicht die Anordnung der Stützstrukturen zumindest teilweise oberhalb und/oder zumindest teilweise unterhalb einer Prozesseinheit, insbesondere vollständig oberhalb und/oder unterhalb einer Prozesseinheit, das Design einer individuellen Plattenbreite für jede der Wärmeaustauscher-Platten. Dadurch kann eine insbesondere runde Querschnittsfläche (Kreisfläche) des Druckmantels am effektivsten ausgenutzt werden.

Eine Ausführungsform des erfindungsgemäßen Reaktors ist dadurch gekennzeichnet, dass die erste Prozesseinheit eine als zumindest teilweise unterhalb der ersten Prozesseinheit angeordnete und als Abstützung ausgebildete Stützstruktur aufweist und die zweite Prozesseinheit eine als zumindest teilweise oberhalb der zweiten Prozesseinheit angeordnete und als Aufhängung ausgebildete Stützstruktur aufweist.

Gemäß dieser Ausführungsform ist die erste, also obere Prozesseinheit über eine als Abstützung ausgebildete Stützstruktur mit dem Druckmantel verbunden. Zusätzlich ist die zweite, also untere Prozesseinheit über eine als Aufhängung ausgebildete Stützstruktur mit dem Druckmantel verbunden. Jede der Prozesseinheiten verfügt somit über eine dedizierte Stützstruktur. Dies führt in bestimmten Reaktorkonfigurationen zu einer vorteilhaften zweiteiligen Abtragung der Lasten der Prozesseinheiten innerhalb des Druckmantels.

Vorzugsweise ist die Abstützung im unteren Bereich der ersten Prozesseinheit, also am Sumpfbereich der ersten Prozesseinheit, angeordnet und stellt in diesem Bereich eine mechanische Verbindung der ersten Prozesseinheit mit dem Druckmantel her. Auch die Elemente zur Ausbildung der mechanischen Verbindung zwischen Prozesseinheit und Druckmantel sind entsprechend und vorzugsweise im unteren Bereich oder am Sumpfbereich der ersten Prozesseinheit angeordnet.

Vorzugsweise ist die Aufhängung im oberen Bereich der zweiten Prozesseinheit, also am Kopfbereich der zweiten Prozesseinheit, angeordnet und stellt in diesem Bereich eine mechanische Verbindung der zweiten Prozesseinheit mit dem Druckmantel her. Auch die Elemente zur Ausbildung der mechanischen Verbindung zwischen Prozesseinheit und Druckmantel sind entsprechend und vorzugsweise im oberen Bereich oder am Kopfbereich der zweiten Prozesseinheit angeordnet.

Aufgrund möglicher unterschiedlicher verwendeter Materialien für den Druckmantel und die Platten der ersten und zweiten Prozesseinheit und allgemein unterschiedlichen Temperaturen zwischen Prozesseinheiten und Druckmantel kann es im Reaktorbetrieb zu unterschiedlichen relativen Dehnungen dieser beiden Elemente kommen, was zu unterschiedlichem Längenwachstum dieser Elemente nach oben und unten führen kann. In diesem Fall ist es von Vorteil, wenn sich die Platten der ersten Prozesseinheit und gegebenenfalls weitere mit diesen Platten mechanisch verbundene Bauteile frei nach oben ausdehnen können. Ferner ist es von Vorteil, wenn sich die Platten der zweiten Prozesseinheit und gegebenenfalls weitere mit diesen Platten mechanisch verbundene Bauteile frei nach unten ausdehnen können. Die beiden Prozesseinheiten wachsen somit mit steigender Temperatur in entgegengesetzter Richtung entsprechend nach oben (erste Prozesseinheit) und nach unten (zweite Prozesseinheit). Dabei liegen die mechanischen Fixpunkte der Aufhängung und Abstützung am Druckmantel nahe beieinander. Spannungen resultierend aus unterschiedlicher Temperaturausdehnung zwischen diesen Fixpunkten werden dadurch klein gehalten, da der Abstand zwischen dem Fixpunkt der oberen Abstützung und dem Fixpunkt der unteren Aufhängung kurz ist.

Eine Ausführungsform des erfindungsgemäßen Reaktors ist dadurch gekennzeichnet, dass die erste Prozesseinheit eine als zumindest teilweise oberhalb der ersten Prozesseinheit angeordnete und als Aufhängung ausgebildete Stützstruktur aufweist, und wobei die erste und die zweite Prozesseinheit über ein Verbindungselement mechanisch miteinander verbunden sind.

Insbesondere stellt das Verbindungselement keine mechanische Verbindung zwischen der ersten Prozesseinheit und dem Druckmantel her, und keine mechanische Verbindung zwischen der zweiten Prozesseinheit und dem Druckmantel her.

Gemäß dieser Ausführungsform ist die erste, also obere Prozesseinheit über eine als Aufhängung ausgebildete Stützstruktur mit dem Druckmantel verbunden. Somit verfügt lediglich die erste Prozesseinheit über eine dedizierte Stützstruktur. Dies führt in bestimmten Reaktorkonfiguration zu einer vorteilhaften einteiligen Abtragung der Lasten beider Prozesseinheiten nach unten innerhalb des Druckmantels.

Vorzugsweise ist die Aufhängung im oberen Bereich der ersten Prozesseinheit, also am Kopfbereich der ersten Prozesseinheit, angeordnet und stellt in diesem Bereich eine mechanische Verbindung der ersten Prozesseinheit mit dem Druckmantel her. Auch die Elemente zur Ausbildung der mechanischen Verbindung zwischen Prozesseinheit und Druckmantel sind entsprechend und vorzugsweise im oberen Bereich oder am Kopfbereich der ersten Prozesseinheit angeordnet.

Gemäß dieser Ausführungsform mit Aufhängung als Stützstruktur für die erste Prozesseinheit und Verbindungselement zwischen erster und zweiter Prozesseinheit kann sich die gesamte Konstruktion aufweisend die Wärmeübertrager-Platten der ersten und zweiten Prozesseinheit und weitere damit mechanisch verbundene Bauteile bei Erhöhung der Temperatur im Reaktorbetrieb frei nach unten ausdehnen.

Bei dieser rein hängenden Konstruktion der ersten und zweiten Prozesseinheit innerhalb des Druckmantels ist die Fixierung am Druckmantel mechanisch aufwändiger, da zusätzliches Gewicht auf die mechanische Verbindung wirkt.

Insbesondere bei Verwendung von siedendem Kesselspeisewasser als Kühlmedium CM1 für die erste Prozesseinheit kann diese Ausführung jedoch von Vorteil sein. In der ersten Prozesseinheit, welche als Reaktorstufe RS1 konfiguriert ist, werden bei bestimmten Reaktorkonfigurationen große Mengen Dampf auf der Platteninnenseite produziert, wobei der Dampf die erste Prozesseinheit aufgrund der Führung des Kühlmittels CM1 von unten nach oben am Kopfbereich der ersten Prozesseinheit verlässt. Die Kühlmedienzufuhrleitung hat dann tendenziell einen kleineren Querschnitt als die dampfführende Kühlmedienabfuhrleitung. Ein kleinerer Querschnitt ist verbunden mit einer kleineren Wanddickte der Zufuhrleitung, wodurch die Zufuhrleitung leichter flexibel ausgeführt werden kann als die Abfuhrleitung. Entsprechend ist es von Vorteil, den Sumpfbereich der ersten Prozesseinheit flexibel auszuführen und den Kopfbereich dafür starr. Ersteres wird durch das flexible, nicht mit dem Druckmantel mechanisch verbundene Verbindungselement zwischen erster und zweiter Prozesseinheit realisiert, letzteres durch die im Kopfbereich der ersten Prozesseinheit als Aufhängung realisierte Stützstruktur.

Um die Spannungen weiter zu reduzieren, kann die Kühlmedienzufuhrleitung parallel zu den Platten der ersten Prozesseinheit geführt werden. Dabei ist der Kühlmedieneintrittstutzen für das Kühlmedium CM1 im Kopfbereich der ersten Prozesseinheit angeordnet, und die Leitung wird anschließend vorzugsweise senkrecht nach unten geführt.

Eine Ausführungsform des erfindungsgemäßen Reaktors ist dadurch gekennzeichnet, dass die zweite Prozesseinheit eine als zumindest teilweise unterhalb der zweiten Prozesseinheit angeordnete und als Abstützung ausgebildete Stützstruktur aufweist, und wobei die erste und die zweite Prozesseinheit über ein Verbindungselement mechanisch miteinander verbunden sind.

Insbesondere stellt das Verbindungselement keine mechanische Verbindung zwischen der ersten Prozesseinheit und dem Druckmantel her, und keine mechanische Verbindung zwischen der zweiten Prozesseinheit und dem Druckmantel her.

Gemäß dieser Ausführungsform ist die zweite, also untere Prozesseinheit über eine als Abstützung ausgebildete Stützstruktur mit dem Druckmantel verbunden. Somit verfügt lediglich die zweite Prozesseinheit über eine dedizierte Stützstruktur. Dies führt in bestimmten Reaktorkonfiguration zu einer vorteilhaften einteiligen Abtragung der Lasten beider Prozesseinheiten nach oben innerhalb des Druckmantels.

Vorzugsweise ist die Abstützung im unteren Bereich der zweiten Prozesseinheit, also im Sumpfbereich der zweiten Prozesseinheit, angeordnet und stellt in diesem Bereich eine mechanische Verbindung der zweiten Prozesseinheit mit dem Druckmantel her. Auch die Elemente zur Ausbildung der mechanischen Verbindung zwischen Prozesseinheit und Druckmantel sind entsprechend und vorzugsweise im unteren Bereich oder im Sumpfbereich der zweiten Prozesseinheit angeordnet.

Gemäß dieser Ausführungsform mit Abstützung als Stützstruktur für die zweite Prozesseinheit und Verbindungselement zwischen erster und zweiter Prozesseinheit kann sich die gesamte Konstruktion aufweisend die Wärmeübertrager-Platten der ersten und zweiten Prozesseinheit und weitere damit mechanisch verbundene Bauteile bei Erhöhung der Temperatur im Reaktorbetrieb frei nach oben ausdehnen.

Eine Ausführungsform des erfindungsgemäßen Reaktors ist dadurch gekennzeichnet, dass der Methanolsynthese-Reaktor einen sich durch den Druckmantel hindurch erstreckenden Synthesegaseintrittstutzen aufweist, wobei der Synthesegaseintrittstutzen fluidisch mit den Platteninnenräumen der zweiten Prozesseinheit verbunden ist und zum Einleiten des Synthesegasstroms in die zweite Prozesseinheit konfiguriert ist.

Diese Ausführungsform ist für den Fall relevant, dass der Synthesegas-Strom als Kühlmittel auf der Platteninnenseite der zweiten Prozesseinheit verwendet wird. Der Synthesegaseintrittstutzen ist dann vorzugsweise am Sumpfbereich der zweiten Prozesseinheit angeordnet und erstreckt sich vorzugsweise in horizontaler Richtung durch den Druckmantel hindurch.

Wird die zweite Prozesseinheit nicht mit Synthesegas als Kühlmittel CM2 betrieben, ist der Synthesegaseintrittstutzen vorzugsweise am Kopfbereich der ersten Prozesseinheit angeordnet und erstreckt sich vorzugsweise in horizontaler Richtung durch den Druckmantel hindurch.

Eine Ausführungsform des erfindungsgemäßen Reaktors ist dadurch gekennzeichnet, dass die zweite Prozesseinheit in ihrem Sumpfbereich ein Verteilersystem aufweist, welches fluidisch mit dem Synthesegaseintrittstutzen und den Platteninnenräumen der zweiten Prozesseinheit verbunden ist und zum Verteilen des über den Synthesegaseintrittstutzen eintretenden Synthesegas-Stroms auf die Platteninnenräume der zweiten Prozesseinheit konfiguriert ist.

Eine Ausführungsform des erfindungsgemäßen Reaktors ist dabei dadurch gekennzeichnet, dass die zweite Prozesseinheit in ihrem Kopfbereich ein Sammlersystem umfasst, welches fluidisch mit den Platteninnenräumen der zweiten Prozesseinheit verbunden ist und zum Sammeln des aus den Platteninnenräumen der zweiten Prozesseinheit austretenden, vorgewärmten Synthesegas-Stroms konfiguriert ist.

In Bezug auf den Kühlmedien-Strom CM2 umfasst die zweite Prozesseinheit vorzugsweise in ihrem Kopfbereich ein Sammlersystem, welches fluidisch mit den Platteninnenräumen der zweiten Prozesseinheit verbunden ist und zum Sammeln des aus den Platteninnenräumen der zweiten Prozesseinheit austretenden Kühlmedien-Stroms CM2 konfiguriert ist.

Eine Ausführungsform des erfindungsgemäßen Reaktors ist dadurch gekennzeichnet, dass die erste Prozesseinheit in ihrem Kopfbereich ein Verteilersystem umfasst, welches fluidisch mit dem Sammlersystem der zweiten Prozesseinheit verbunden ist und zum Verteilen des vorgewärmten Synthesegases auf die Plattenzwischenräume der ersten Prozesseinheit konfiguriert ist.

Wird der Synthesegas-Strom nicht als Kühlmedien-Strom CM2 in der zweiten Prozesseinheit verwendet, so umfasst die erste Prozesseinheit in ihrem Kopfbereich vorzugsweise ein Verteilersystem, welches zum Verteilen des Synthesegases des Synthese-gas-Stroms auf die Plattenzwischenräume der ersten Prozesseinheit konfiguriert ist.

Eine Ausführungsform des erfindungsgemäßen Reaktors ist dadurch gekennzeichnet, dass das Sammlersystem der zweiten Prozesseinheit und das Verteilersystem der ersten Prozesseinheit über eine die erste Prozesseinheit umgehende und zumindest teilweise innerhalb des Druckmantels verlaufende Leitung verbunden sind.

Diese Ausführungsform ist dann von Relevanz, wenn der Synthesegas-Stroms als Kühlmedien-Strom CM2 in der zweiten Prozesseinheit verwendet wird und das Sammlersystem der ersten Prozesseinheit und das Verteilersystem der ersten Prozesseinheit durch eine entsprechende Leitung miteinander verbunden werden müssen. Es ist dann von Vorteil, diese Leitung so zu legen, dass diese die erste Prozesseinheit zwischen dem Druckmantel und dieser ersten Prozesseinheit umgeht und dabei insbesondere parallel zu den Platten der ersten Prozesseinheit verläuft.

Eine Ausführungsform des erfindungsgemäßen Reaktors ist dadurch gekennzeichnet, dass der Methanolsynthese-Reaktor einen sich durch den Druckmantel hindurch erstreckenden Kühlmedieneintrittstutzen aufweist, wobei der Kühlmedieneintrittstutzen fluidisch mit den Platteninnenräumen der ersten Prozesseinheit verbunden ist und zum Einleiten des Kühlmedien-Strom CM1 in die erste Prozesseinheit konfiguriert ist.

Eine Ausführungsform des erfindungsgemäßen Reaktors ist dabei dadurch gekennzeichnet, dass die erste Prozesseinheit in ihrem Sumpfbereich ein Verteilersystem aufweist, welches fluidisch mit dem Kühlmedieneintrittstutzen und den Platteninnenräumen der ersten Prozesseinheit verbunden ist und zum Verteilen des über den Kühlmedieneintrittstutzen eintretenden Kühlmedien-Strom CM1 auf die Platteninnenräume der ersten Prozesseinheit konfiguriert ist.

Der Kühlmedien-Strom CM1 umfasst in Bezug auf die erste Prozesseinheit vorzugsweise siedendes Kesselspeisewasser.

Eine Ausführungsform des erfindungsgemäßen Reaktors ist dadurch gekennzeichnet, dass der Methanolsynthese-Reaktor einen sich durch den Druckmantel hindurch erstreckenden Kühlmedienaustrittstutzen aufweist, wobei der Kühlmedienaustrittstutzen fluidisch mit den Platteninnenräumen der ersten Prozesseinheit verbunden ist und zum Ausleiten des Kühlmedien-Strom CM1 aus der ersten Prozesseinheit konfiguriert ist.

Bei Verwendung von siedendem Kesselspeisewasser für den Kühlmedien-Strom CM1 umfasst der ausgeleitete Kühlmedien-Strom CM1 insbesondere Dampf.

Eine Ausführungsform des erfindungsgemäßen Reaktors ist dabei dadurch gekennzeichnet, dass die erste Prozesseinheit in ihrem Kopfbereich ein Sammlersystem aufweist, welches fluidisch mit dem Kühlmedienaustrittstutzen und den Platteninnenräumen der ersten Prozesseinheit verbunden ist und zum Sammeln des aus den Platteninnenräumen der ersten Prozesseinheit austretenden Kühlmedien-Strom CM1 konfiguriert ist.

Eine Ausführungsform des erfindungsgemäßen Reaktors ist dadurch gekennzeichnet, dass der Methanolsynthese-Reaktor einen sich durch den Druckmantel hindurch erstreckenden Produktstromaustrittstutzen umfasst, wobei der Produktstromaustrittstutzen fluidisch mit den Plattenzwischenräumen der zweiten Prozesseinheit verbunden ist und zum Ausleiten des methanolhaltigen Produkstroms RP1 und/oder des methanolhaltigen Produktstrom RP2 aus der zweiten Prozesseinheit konfiguriert ist.

Der Produktstromaustrittstutzen ist im Falle der Alternative c1 zum Ausleiten des methanolhaltigen Produkstroms RP1 aus der zweiten Prozesseinheit konfiguriert. Der Produktstromaustrittstutzen ist im Falle der Alternative c2 zum Ausleiten des methanolhaltigen Produkstroms RP2 aus der zweiten Prozesseinheit konfiguriert.

Eine Ausführungsform des erfindungsgemäßen Reaktors ist dadurch gekennzeichnet, dass die zweite Prozesseinheit in ihrem Sumpfbereich ein Sammlersystem aufweist, welches fluidisch mit dem Produkstromaustrittstutzen und den Plattenzwischenräumen der zweiten Prozesseinheit verbunden ist und zum Sammeln des aus den Plattenzwischenräumen der zweiten Prozesseinheit austretenden methanolhaltigen Produkstrom RP1 und/oder methanolhaltigen Produktstrom RP2 konfiguriert ist.

Das Sammlersystem ist im Falle der Alternative c1 zum Sammeln des aus den Plattenzwischenräumen der zweiten Prozesseinheit austretenden methanolhaltigen Produkstrom RP1 konfiguriert. Das Sammlersystem ist im Falle der Alternative c2 zum Sammeln des aus den Plattenzwischenräumen der zweiten Prozesseinheit austretenden methanolhaltigen Produkstrom RP2 konfiguriert.

Eine Ausführungsform des erfindungsgemäßen Reaktors ist dadurch gekennzeichnet, dass zwischen der ersten Prozesseinheit und der zweiten Prozesseinheit innerhalb des Druckmantels ein Zwischenraumbereich angeordnet ist, wobei der Zwischenraumbereich fluidisch mit den Plattenzwischenräumen der ersten Prozesseinheit und den Plattenzwischenräumen der zweiten Prozesseinheit verbunden ist und zum Überführen des methanolhaltigen Produktstroms RP1 von der ersten Prozesseinheit in die zweite Prozesseinheit konfiguriert ist.

Dabei kann der Zwischenraumbereich ein Sammlersystem aufweisen, welches fluidisch mit den Plattenzwischenräumen der ersten Prozesseinheit verbunden ist und zum Sammeln des aus den Plattenzwischenräumen der ersten Prozesseinheit austretenden methanolhaltigen Produktstrom RP1 konfiguriert ist.

Ferner kann der Zwischenraumbereich ein Verteilersystem aufweisen, welches fluidisch mit den Plattenzwischenräumen der zweiten Prozesseinheit verbunden ist und zum Verteilen des methanolhaltigen Produktstrom RP1 auf die Plattenzwischenräume der zweiten Prozesseinheit konfiguriert ist.

Das Sammlersystem der ersten Prozesseinheit und das Verteilersystem der zweiten Prozesseinheit des Zwischenraumbereichs sind dabei fluidisch miteinander verbunden.

### Ausführungsbeispiel

Die Erfindung wird im Folgenden durch Ausführungsbeispiele näher erläutert. In der folgenden detaillierten Beschreibung wird auf die beigefügten Zeichnungen verwiesen, die einen Teil der Ausführungsbeispiele bilden und in welcher illustrativ spezifische Ausführungsformen der Erfindung dargestellt ist. In diesem Zusammenhang wird richtungsspezifische Terminologie wie "oben", "unten", "vorne", "hinten" usw. in Bezug auf die Ausrichtung der beschriebenen Figur verwendet. Da Komponenten von Ausführungsformen in einer Vielzahl von Ausrichtungen positioniert werden können, dient die richtungsspezifische Terminologie zur Veranschaulichung und ist in keiner Weise limitierend.

Die folgende detaillierte Beschreibung ist daher nicht in einem einschränkenden Sinne zu verstehen, und der Schutzumfang der Ausführungsformen wird durch die beigefügten Ansprüche definiert. Die Zeichnungen sind, soweit nicht anders angegeben, nicht maßstabsgetreu.

In der folgenden Beschreibung und in den Zeichnungen sind gleiche Elemente mit gleichen Bezugszeichen bezeichnet. Pfeile illustrieren die Strömungsrichtung der Prozessmedien, also des Synthesegas-Stroms und der methanolhaltigen Produktströme RP1 und RP2, sowie der Kühlmedien CM1 und CM2.

Es zeigen
- Figur 1: eine stark vereinfachte schematische Darstellung eines Methanolsynthese-Reaktors nach Alternative c1 gemäß einem ersten Beispiel der Erfindung,
- Figur 2: eine stark vereinfachte schematische Darstellung eines Methanolsynthese-Reaktors nach Alternative c1 gemäß einem zweiten Beispiel der Erfindung,
- Figur 3: eine stark vereinfachte schematische Darstellung eines Methanolsynthese-Reaktors nach Alternative c2 gemäß einem dritten Beispiel der Erfindung,
- Figur 4: eine stark vereinfachte schematische Darstellung eines Methanolsynthese-Reaktors nach Alternative c2 gemäß einem vierten Beispiel der Erfindung,
- Figur 5: ein messtechnisch und durch eine Simulation ermitteltes typisches Temperaturprofil eines Katalysatorbetts über die Länge eines Methanol-Reaktors.

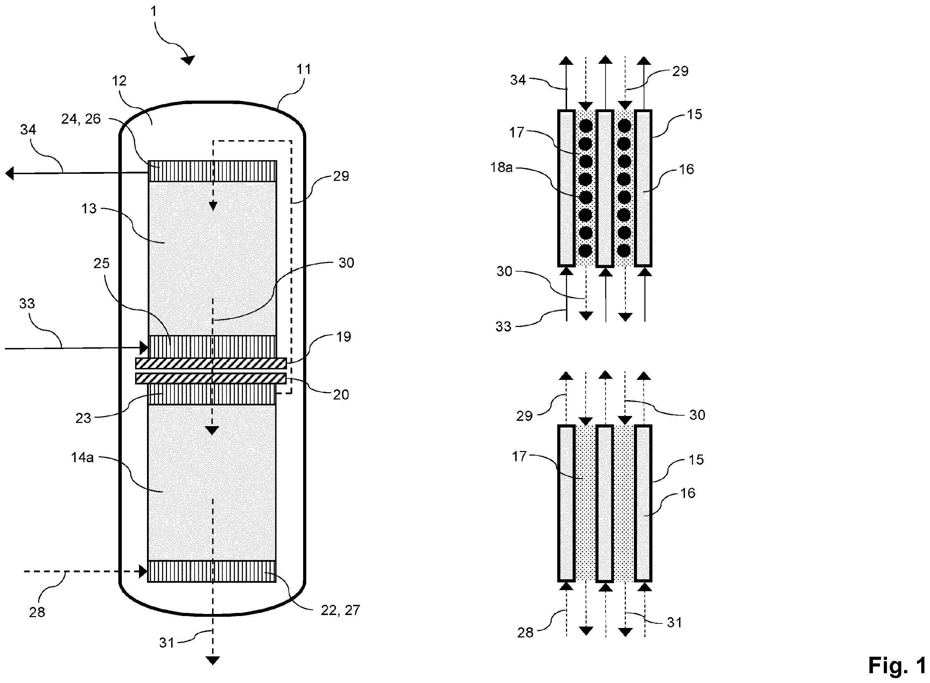

Figur 1 zeigt eine stark vereinfachte schematische Darstellung (Prinzipskizze) eines Methanolsynthese-Reaktors 1 gemäß einem ersten Beispiel der Erfindung. Gemäß Alternative c1 weist der Methanolsynthese-Reaktor 1 eine erste Prozesseinheit 13 auf, welche als Reaktorstufe RS1 ausgestaltet ist. Weiterhin und gemäß Alternative c1 weist der Methanolsynthese-Reaktor 1 eine zweite Prozesseinheit 14a auf, welche als Kühlstufe ausgestaltet ist.

Der Methanolsynthese-Reaktor 1 ist auf der linken Seite von Figur 1 in einer Frontansicht dargestellt. Figur 1 zeigt weiterhin eine Seitenansicht eines Teils der ersten Prozesseinheit 13 rechts oben sowie eine Seitenansicht eines Teils der zweiten Prozesseinheit 14a rechts unten.

Der Methanolsynthese-Reaktor 1 gemäß Figur 1 weist einen Druckmantel 11 auf, in dessen Innenraum 12 eine erste Prozesseinheit 13 und eine zweite Prozesseinheit 14a angeordnet sind. Die erste Prozesseinheit 13 ist in einem oberen Teil des Innenraums 12 angeordnet, während die zweite Prozesseinheit 14a in einem unteren Teil des Innenraums 12 und unterhalb der ersten Prozesseinheit 13 angeordnet ist. Beide Prozesseinheiten 13 und 14a sind fluidisch, beispielsweise über entsprechende Rohrleitungen von Verteiler- und Sammlersystemen, miteinander verbunden (nicht gezeigt).

Der Methanolsynthese-Reaktor 1 ist als einstufiger Reaktor mit einer Reaktorstufe RS1 und einer Kühlstufe konfiguriert. Dabei wird die Reaktorstufe RS1 von der ersten Prozesseinheit 13 gebildet und die Kühlstufe von der zweiten Prozesseinheit 14a. Als Kühlmedien-Strom CM1 in der ersten Reaktorstufe wird siedendes Kesselspeisewasser verwendet. Als Kühlmedien-Strom CM2 in der Kühlstufe wird der Synthesegas-Strom verwendet, welcher auch in der Reaktorstufe RS1 zur Umsetzung zu Methanol an einem Katalysatorbett CB1 verwendet wird. Der Synthesegas-Strom wird in der zweiten Prozesseinheit 14a vorgewärmt und kann anschließend als vorgewärmter Synthesegas-Strom in die erste Prozesseinheit 13 zur Umsetzung zu Methanol eingeleitet werden.

Die erste Prozesseinheit 13 ist als Plattenwärmeübertrager ausgestaltet, wobei der Plattenwärmeübertrager eine Vielzahl von durchströmbaren (Wärmeübertrager-)Platten 15 aufweist, welche als Kissenplatten ausgestaltet sind (Kissenplatten-Struktur nicht dargestellt). Die Platten sind vertikal angeordnet und werden entsprechend in vertikaler Richtung durchströmt. Die Platten 15 weisen jeweils einen durchströmbaren Platteninnenraum 16 auf, welcher von dem Kühlmedien-Strom CM1 33 von unten nach oben durchströmt wird. Dabei tritt der Strom des frischen Kühlmediums CM1 33 von unten in eine Platte 15 ein und tritt als Strom von verbrauchtem Kühlmedium CM1 34 oben aus der betreffenden Platte 15 aus. Bei dem frischen Kühlmedien-Strom CM1 33 handelt es sich um siedendes Kesselspeisewasser. Bei dem verbrauchten Kühlmedien-Strom CM1 34 handelt es sich um Wasserdampf.

Zwei nebeneinander angeordnete Platten 15 der ersten Prozesseinheit 13 sind so voneinander beabstandet, dass sie einen Plattenzwischenraum 17 bilden. In den Plattenzwischenräumen 17 sind Pellets eines Methanolsynthese-Katalysators angeordnet, angedeutet durch die schwarzen Punkte. Die Gesamtheit der Katalyator-Pellets bilden das Katalysatorbett CB1 18a. Ein vorgewärmter Synthesegas-Strom 29 tritt von oben in die Plattenzwischenräume 17 ein und wird am Katalysatorbett CB1 18a zumindest teilweise in einer exothermen Reaktion zu einem Gemisch aus Methanol und Wasser (Rohmethanol) umgesetzt. Die Plattenzwischenräume werden dabei im Gegenstrom durch den Kühlmedien-Strom CM1 33 gekühlt beziehungsweise dadurch die Temperatur in den Plattenzwischenräumen kontrolliert. Im Rahmen dieser exothermen Reaktion wird ein Produktstrom RP1 30 gebildet, welcher zumindest Methanol, Wasser und nicht umgesetztes Synthesegas (Restgas) beinhaltet. Dieser Produktstrom RP1 30 tritt unten aus den Plattenzwischenräumen 17 aus.

Auch die zweite Prozesseinheit 14a ist als Plattenwärmeübertrager ausgestaltet, wobei der Plattenwärmeübertrager eine Vielzahl von durchströmbaren (Wärmeübertrager- )Platten 15 aufweist, welche als Kissenplatten ausgestaltet sind (Kissenplatten-Struktur nicht dargestellt). Die Platten sind vertikal angeordnet und werden entsprechend in vertikaler Richtung durchströmt. Die Platten 15 der zweiten Prozesseinheit weisen jeweils einen durchströmbaren Platteninnenraum 16 auf, welcher von dem Kühlmedien-Strom CM2 28 von unten nach oben durchströmt wird. Dabei tritt der Strom des frischen Kühlmediums CM2 28 von unten in die Platte 15 ein und tritt als Strom von verbrauchtem Kühlmedium CM2 29 oben aus der Platte 15 aus. Bei dem frischen Kühlmedien-Strom CM2 28 handelt es sich um den Synthesegas-Strom. Bei dem verbrauchten Kühlmedien-Strom CM2 29 handelt es sich um den vorgewärmten Synthesegas-Strom.

Zwei nebeneinander angeordnete Platten 15 der zweiten Prozesseinheit 14a sind so voneinander beabstandet, dass sie einen Plattenzwischenraum 17 bilden. In den Plattenzwischenräumen 17 der zweiten Prozesseinheit ist im Gegensatz zu den Plattenzwischenräumen der ersten Prozesseinheit 13 kein Methanolsynthese-Katalysator angeordnet, da die zweite Prozesseinheit alleine der Kühlung und gegebenenfalls Kondensation des methanolhaltigen Produktstroms RP1 30 dient. Die von dem methanolhaltigen Produktstrom RP1 30 von oben nach unten durchströmten Plattenzwischenräume 17 werden im Gegenstrom durch den Kühlmedien-Strom CM2 28 gekühlt, wodurch sich der zunächst heiße methanolhaltige Produktstrom RP1 30 zum kalten methanolhaltigen Produktstrom RP1 31 abkühlt. Je nach Auslegung der zweiten Prozesseinheit 14a kann der methanolhaltige Produktstrom RP1 31 dabei in lediglich abgekühlter jedoch noch gasförmiger Form, in teilkondensierter oder vollständig kondensierter Form in Bezug auf die kondensierbaren Anteile anfallen.

Wie auf der rechten Seite der Figur 1 dargestellt, sind die durchströmbaren Platten 15 mit ihren Platteninnenräumen 16 und die Plattenzwischenräume 17 der ersten und zweiten Prozesseinheit 13 und 14a jeweils zueinander fluchtend hintereinander angeordnet.

Mit anderen Worten, die Platten und Plattenzwischenräume der ersten und zweiten Prozesseinheit 13 und 14a befinden sich jeweils hintereinander auf einer gemeinsamen Längsachse.

Die erste Prozesseinheit 13 des Methanolsynthese-Reaktors 1 ist über eine Stützstruktur 19 mechanisch mit dem Druckmantel 11 verbunden, wobei also die Stützstruktur 19 eine mechanische Verbindung zwischen der ersten Prozesseinheit und dem Druckmantel 11 ausbildet (Verbindung zum Druckmantel liegt in der Zeichenebene). Die Stützstruktur 19 ist dabei als Abstützung ausgestaltet und zumindest teilweise unterhalb der ersten Prozesseinheit 13 angeordnet. Im Gegensatz zu einer Anordnung seitlich der ersten Prozesseinheit 13 weist diese Anordnung den Vorteil auf, dass der Durchmesser des Methanolsynthese-Reaktors 1 nicht signifikant beeinflusst wird, insbesondere nicht signifikant vergrößert wird. Durch die Ausbildung der Stützstruktur 19 als Abstützung kann sich die erste Prozesseinheit frei nach oben ausdehnen. Dies betrifft insbesondere das Paket der Wärmeübertrager-Platten und damit mechanisch verbundene Bauteile. Mechanisch mit den Wärmeübertrager-Platten verbundene Bauteile sind insbesondere die Verteiler- und Sammlersysteme 24, 25 und 26.

Die zweite Prozesseinheit 14a des Methanolsynthese-Reaktors 1 ist über eine Stützstruktur 20 mechanisch mit dem Druckmantel 11 verbunden, wobei also die Stützstruktur 20 eine mechanische Verbindung zwischen der zweiten Prozesseinheit und dem Druckmantel 11 ausbildet (Verbindung zum Druckmantel liegt in der Zeichenebene). Die Stützstruktur 20 ist dabei als Aufhängung ausgestaltet und zumindest teilweise oberhalb der zweiten Prozesseinheit 14a angeordnet. Im Gegensatz zu einer Anordnung seitlich der zweiten Prozesseinheit 14a weist diese Anordnung den Vorteil auf, dass der Durchmesser des Methanolsynthese-Reaktors 1 nicht signifikant beeinflusst wird, insbesondere nicht signifikant vergrößert wird. Durch die Ausbildung der Stützstruktur 20 als Aufhängung kann sich die zweite Prozesseinheit 14a frei nach unten ausdehnen. Dies betrifft insbesondere das Paket der Wärmeübertrager-Platten und damit mechanisch verbundene Bauteile. Mechanisch mit den Wärmeübertrager-Platten verbundene Bauteile sind insbesondere die Verteiler- und Sammlersysteme 22, 23 und 27.

Die Strömungsführung im erfindungsgemäßen Reaktor 1 wird im Folgenden erläutert. Der Synthesegas-Strom 28 tritt über einen Synthesegaseintrittstutzen (nicht gezeigt), welcher sich in horizontaler Richtung durch den Druckmantel 11 hindurch erstreckt, in den Methanolsynthese-Reaktor 1 ein. Der Synthesegas-Strom 28 wird anschließend über ein Verteilersystem 22 (vereinfacht und aus Gründen der Übersichtlichkeit als Teil eines Blocks mit den Komponenten 22, 27 dargestellt) auf die einzelnen Platteninnenräume 16 der ersten Prozesseinheit verteilt. Das Synthesegas strömt anschließend von unten nach oben durch die Platteninnenräume 16 hindurch und kühlt dabei im Gegenstrom den Produktstrom RP1 30 auf der Seite der Plattenzwischenräume 17. Der Synthesegas-Strom 28 erfüllt dabei die Funktion des Kühlmedien-Stroms CM2. Der sich dabei bildende vorgewärmte Synthesegas-Strom 29 oder verbrauchter Kühlmedien-Strom CM2 wird in einem Sammlersystem 23 zusammengeführt und über eine Bypass-Leitung (angedeutet durch die gestrichelte Linie) zum Kopf der ersten Prozesseinheit geführt. Dort tritt der vorgewärmte Synthesegas-Strom 29 in ein Verteilersystem 24 (vereinfacht und aus Gründen der Übersichtlichkeit als Teil eines Blocks mit den Komponenten 24, 26 dargestellt) ein, welches das Synthese-Gas auf die Plattenzwischenräume 17 der ersten Prozesseinheit verteilt. In den Plattenzwischenräumen 17 der ersten Prozesseinheit reagiert das vorgewärmte Synthesegas am Methanolsynthese-Katalysator des Katalysatorbetts zu Methanol und Wasser und bildet damit den Produktstrom RP1 30, welcher auch nicht umgesetztes Synthesegas (Restgas) umfasst. Der Produktstrom RP1 30 strömt von oben nach unten und wird im Sumpfbereich der ersten Prozesseinheit 13 aus dieser abgezogen, durchströmt einen Zwischenraumbereich (nicht gezeigt) und tritt anschließend zum Abkühlen in die Plattenzwischenräume 17 der zweiten Prozesseinheit 14a ein. Der abgekühlte Produktstrom RP1 31 wird mit Hilfe eines Sammlersystems 27 (vereinfacht und aus Gründen der Übersichtlichkeit als Teil eines Blocks mit den Komponenten 22, 27 dargestellt) zusammengeführt und über einen Produktstromaustrittstutzen (nicht gezeigt) aus dem Reaktor ausgeleitet. Das so erhältliche Rohprodukt wird anschließend einer weiteren Behandlung (zum Beispiel weitere Kühlung und Gas-Flüssigkeits-Trennung) und Aufarbeitung (zum Beispiel Destillation) zugeführt.

Der Zwischenraumbereich zwischen erster und zweiter Prozesseinheit kann ebenfalls ein entsprechendes Sammlersystem für den Produktstrom RP1 im Sumpfbereich der ersten Prozesseinheit 13 und ein entsprechendes Verteilersystem für den Produktstrom RP1 am Kopfbereich der zweiten Prozesseinheit 14a aufweisen.

Der frische Kühlmedien-Strom CM1 33 tritt über einen horizontal angeordneten Kühlmedieneintrittstutzen (nicht gezeigt), welcher sich durch den Druckmantel 11 hindurch erstreckt, in den Reaktor ein und wird über ein Verteilersystem 25 auf die Platteninnenräume 16 der ersten Prozesseinheit 13 verteilt. Das für den Kühlmedien-Strom CM1 33 eingesetzte siedende Kesselspeisewasser verdampft dabei, wodurch eine Kühlung des sich auf den Plattenzwischenräumen bildenden Produktstroms RP1 erfolgt. Der Dampf oder verbrauchte Kühmedien-Strom CM1 34 wird in einem Sammlersystem 26 (vereinfacht und aus Gründen der Übersichtlichkeit als Teil eines Blocks mit den Komponenten 24, 26 dargestellt) zusammengeführt und über einen horizontal angeordneten und sich durch den Druckmantel hindurch erstreckenden Kühlmedienaustrittstutzen aus dem Reaktor 1 ausgeleitet.

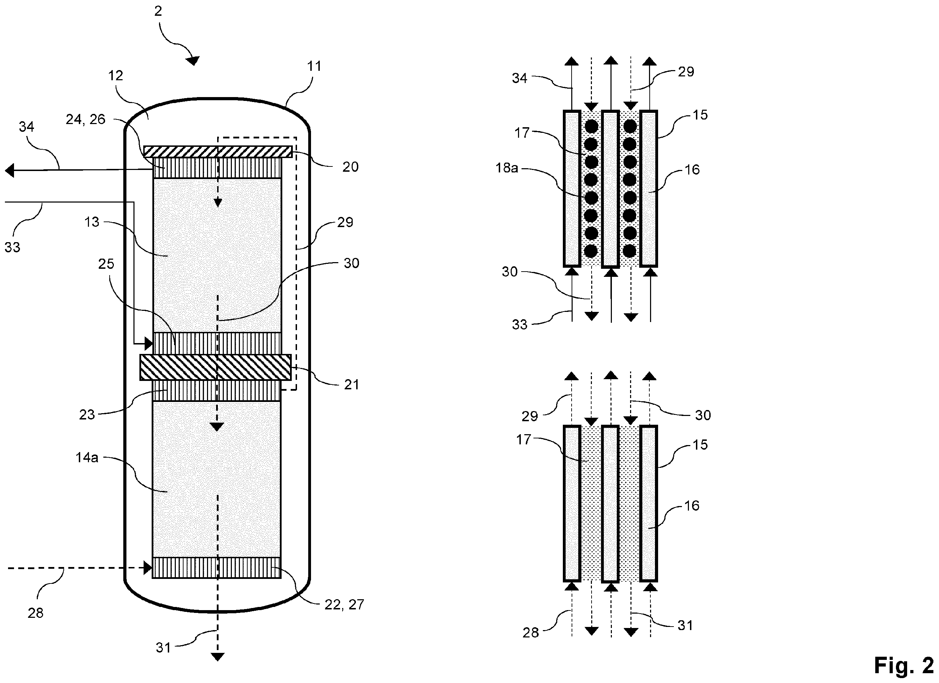

Die Beispiele gemäß Figur 2 bis 4 werden im Folgenden nicht nochmals im Detail erläutert. Es wird lediglich auf Unterschiede zum Beispiel gemäß Figur 1 eingegangen.

Figur 2 zeigt eine stark vereinfachte schematische Darstellung (Prinzipskizze) eines Methanolsynthese-Reaktors 2 gemäß einem zweiten Beispiel der Erfindung. Gemäß Alternative c1 weist auch der Methanolsynthese-Reaktor 2 eine erste Prozesseinheit 13 auf, welche als Reaktorstufe RS1 ausgestaltet ist. Weiterhin und gemäß Alternative c1 weist der Methanolsynthese-Reaktor 2 eine zweite Prozesseinheit 14a auf, welche als Kühlstufe ausgestaltet ist.

Der Methanolsynthese-Reaktor 2 gemäß Figur 2 unterscheidet sich von dem Methanolsynthese-Reaktor 1 gemäß Figur 1 maßgeblich durch die Art der mechanischen Fixierung der ersten und zweiten Prozesseinheiten 13 und 14a. Ansonsten ist auch der Methanolsynthese-Reaktor 2 als einstufiger Reaktor mit einer als wassergekühlten Reaktorstufe RS1 ausgestalteten ersten Prozesseinheit 13 und einer als gasgekühlten Kühlstufe 14a ausgestalteten zweiten Prozesseinheit 14a konfiguriert.

Der Methanolsynthese-Reaktor 2 gemäß dem Beispiel der Figur 2 weist nur eine mechanische Stützstruktur 20 auf, welche die erste Prozesseinheit 13 mechanisch mit dem Druckmantel 11 verbindet. Die Stützstruktur 20 bildet also eine mechanische Verbindung zwischen der ersten Prozesseinheit 13 und dem Druckmantel 11 aus (Verbindung zum Druckmantel liegt in der Zeichenebene). Diese Stützstruktur 20 ist als Aufhängung ausgestaltet und zum indest teilweise oberhalb der ersten Prozesseinheit 13 angeordnet. Im Gegensatz zu einer Anordnung seitlich der ersten Prozesseinheit 13 weist diese Anordnung wie bereits in Bezug auf das Beispiel gemäß Figur 1 erwähnt den Vorteil auf, dass der Durchmesser des Methanolsynthese-Reaktors 2 nicht signifikant beeinflusst wird, insbesondere nicht signifikant vergrößert wird.

Im Gegensatz zum Beispiel gemäß Figur 1 ist die zweite Prozesseinheit 14a nicht mechanisch über eine Stützstruktur mit dem Druckmantel 11 verbunden. Vielmehr sind die erste Prozesseinheit 13 und die zweite Prozesseinheit 14a über ein Verbindungselement 21 mechanisch miteinander verbunden. Das Verbindungselement 21 bildet jedoch keine mechanische Verbindung zum Druckmantel 11 aus. Dadurch können sich die erste Prozesseinheit 13 und die zweite Prozesseinheit 14a frei nach unten ausdehnen. Diese Ausführungsform weist insbesondere bei großen Dampfmengen bezüglich des Kühlmedienstroms CM1 34 den Vorteil auf, dass die Zuführung des Kühlmedien-Stroms CM1 33 im Gegensatz zur Ausführung gemäß Figur 1 mechanisch flexibler gestaltet werden kann.

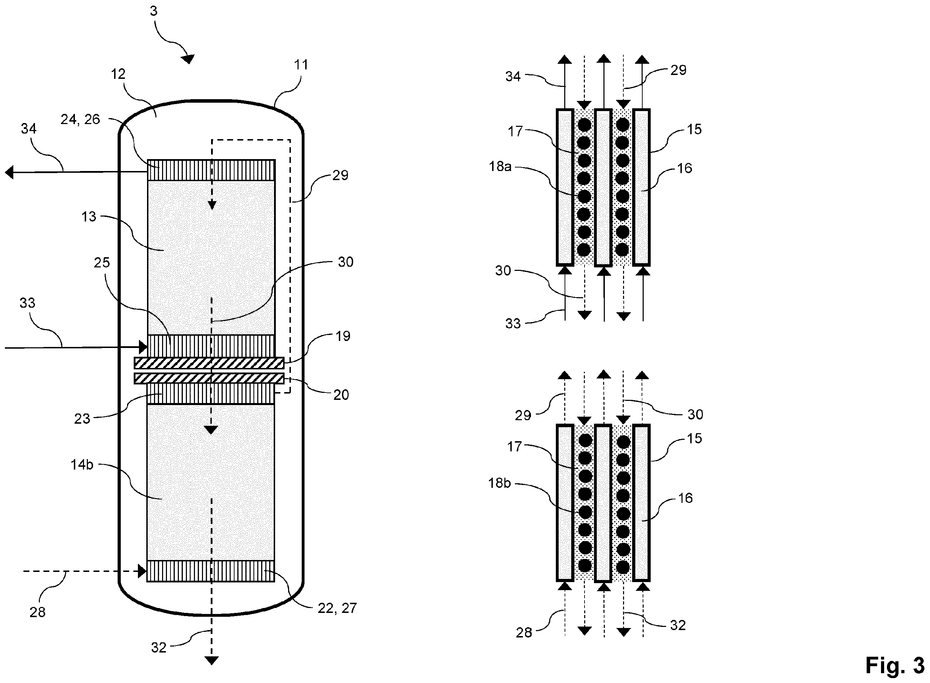

Figur 3 zeigt eine stark vereinfachte schematische Darstellung (Prinzipskizze) eines Methanolsynthese-Reaktors 3 gemäß einem dritten Beispiel der Erfindung. Gemäß Alternative c2 weist der Methanolsynthese-Reaktor 3 eine erste Prozesseinheit 13 auf, welche als Reaktorstufe RS1 ausgestaltet ist. Weiterhin und gemäß Alternative c2 weist der Methanolsynthese-Reaktor 3 eine zweite Prozesseinheit 14b auf, welche als weitere oder zweite Reaktorstufe RS2 ausgestaltet ist.

Im Gegensatz zu den Beispielen des Methanolsynthese-Reaktors gemäß den Figuren 1 und 2 wird der aus der ersten Prozesseinheit 13 austretende methanolhaltige Produktstrom RP1 30 in der zweiten Prozesseinheit 14b nicht nur abgekühlt, sondern vielmehr weiter zu einem methanolhaltigen Produktstrom RP2 32 umgesetzt. Dies wird dadurch ermöglicht, dass der methanolhaltige Produktstrom RP1 30 aufgrund der Einstellung eines thermodynamischen Gleichgewichts nicht umgesetztes Synthesegas (Kohlenstoffoxid(e) und Wasserstoff) aufweist. Dieses nicht umgesetzte Synthesegas oder Restgas wird auf der Plattenzwischenraumseite, das heißt innerhalb der Plattenzwischenräume 17 der zweiten Prozesseinheit 14b am dort angeordneten Katalysatorbett CB2 18b weiter zu Methanol und Wasser umgesetzt. Die Kühlung dieser exothermen Reaktion erfolgt über den im Sumpfbereich der zweiten Prozesseinheit eingespeisten Synthesegas-Strom 28 oder frischen Kühlmedien-Strom CM2 28. Entsprechend ist der Methanolsynthese-Reaktor 3 als zweistufiger Reaktor ausgestaltet, wobei die erste Reaktorstufe (Prozesseinheit 13) als wassergekühlte Reaktorstufe RS1 mit siedendem Kesselspeisewasser als Kühlmedien-Strom CM1 33 und die zweite Reaktorstufe RS2 als gasgekühlte Reaktorstufe mit Synthesegas als Kühlmedien-Strom CM2 28 konfiguriert ist.

Die mechanische Fixierung der ersten und zweiten Prozesseinheit 13 und 14b über die Stützstrukturen 19 und 20 am Druckmantel 11 entspricht dabei der mechanischen Fixierung wie für das Beispiel des Methanolsynthese-Reaktors 1 in Figur 1 gezeigt und beschrieben.

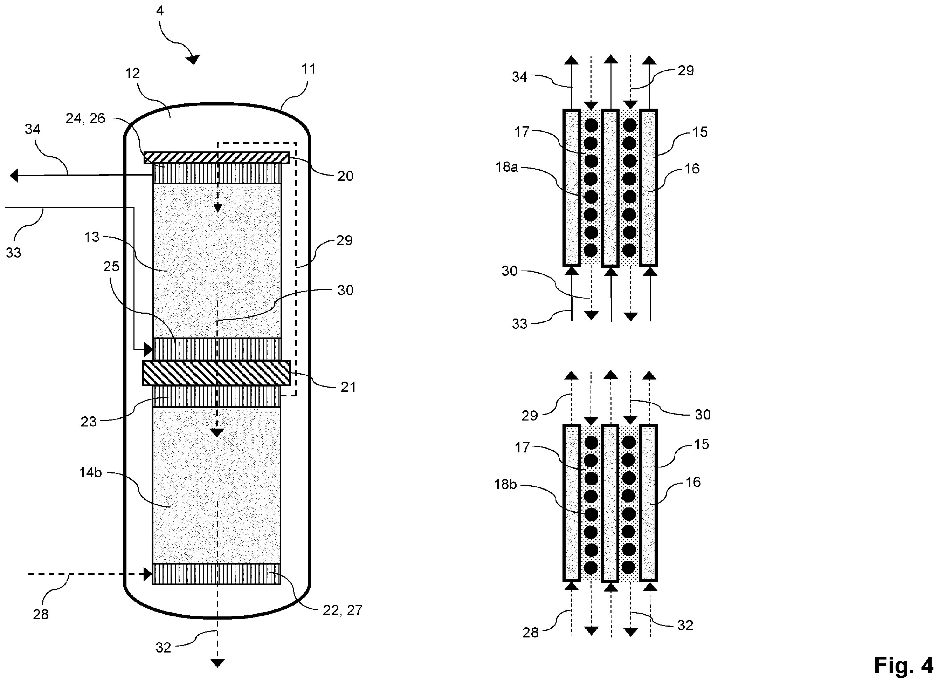

Figur 4 zeigt eine stark vereinfachte schematische Darstellung (Prinzipskizze) eines Methanolsynthese-Reaktors 4 gemäß einem vierten Beispiel der Erfindung. Gemäß Alternative c2 weist der Methanolsynthese-Reaktor 4 eine erste Prozesseinheit 13 auf, welche als Reaktorstufe RS1 ausgestaltet ist. Weiterhin und gemäß Alternative c2 weist der Methanolsynthese-Reaktor 4 eine zweite Prozesseinheit 14b auf, welche als weitere oder zweite Reaktorstufe RS2 ausgestaltet ist. Wie im Beispiel des Reaktors gemäß Figur 3 ist auch der Methanolsynthese-Reaktor 4 als zweistufiger Reaktor ausgestaltet, wobei die erste Reaktorstufe RS1 als wassergekühlte Reaktorstufe und die zweite Reaktorstufe RS2 als gasgekühlte Reaktorstufe konfiguriert ist.

Die mechanische Fixierung der ersten und zweiten Prozesseinheit 13 und 14b über die Stützstruktur 20 am Druckmantel 11 und die Verbindung der ersten und zweiten Prozesseinheit 13 und 14b über das Verbindungselement 21 entspricht dabei der Konfiguration wie für das Beispiel des Methanolsynthese-Reaktors 2 in Figur 2 gezeigt und beschrieben.

### Bezugszeichenliste

- 1, 2, 3, 4: Methanolsynthese-Reaktor
- 11: Druckmantel
- 12: Innenraum
- 13: Erste Prozesseinheit (Reaktorstufe RS1)
- 14a: Zweite Prozesseinheit (Kühlstufe)
- 14b: Zweite Prozesseinheit (Reaktorstufe RS2)
- 15: (Wärmeübertrager-)Platte
- 16: Platteninnenraum
- 17: Plattenzwischenraum
- 18a: Katalysatorbett CB1
- 18b: Katalysatorbett CB2
- 19: Abstützung (Stützstruktur)
- 20: Aufhängung (Stützstruktur)
- 21: Verbindungselement
- 22: Verteilersystem (für Synthesegas-Strom)
- 23: Sammlersystem (für vorgewärmten Synthesegas-Strom)
- 24: Verteilersystem (für vorgewärmten Synthesegas-Strom)
- 25: Verteilersystem (für Kühlmedium)
- 26: Sammlersystem (für Kühlmedium)
- 27: Sammlersystem (für Produktstrom RP1 oder RP2)
- 28: Synthesegas-Strom (Kühlmedien-Strom CM2, frisch)
- 29: Vorgewärmter Synthesegas-Strom (Kühlmedien-Strom CM2, verbraucht)
- 30: Produktstrom RP1
- 31: Abgekühlter Produktstrom RP1
- 32: Produktstrom RP2
- 33: Kühlmedien-Strom CM1, frisch
- 34: Kühlmedien-Strom CM1, verbraucht

## Patentansprüche

1. Methanolsynthese-Reaktor (1, 2, 3, 4) zur Herstellung von Methanol aus einem Synthesegasgemisch, aufweisend
(a) einen Druckmantel (11) mit einem Innenraum (12), wobei in dem Innenraum (12) zumindest zwei fluidisch miteinander verbundene Prozesseinheiten übereinander angeordnet sind;
(b) eine erste Prozesseinheit (13), wobei die erste Prozesseinheit als Reaktorstufe RS1 zur Synthese von Methanol aus einem Synthesegas-Strom (29) an einem Katalysatorbett CB1 (18a) konfiguriert ist, wodurch ein methanolhaltiger Produktstrom RP1 (30) erzeugbar ist, und
die erste Prozesseinheit (13) als Plattenwärmeübertrager ausgestaltet ist, wobei die erste Prozesseinheit (13) dabei eine Vielzahl von vertikal und parallel zueinander angeordneten Platten (15) mit durchströmbaren Platteninnenräumen (16) aufweist, und zwischen benachbarten Platten Plattenzwischenräume (17) ausgebildet sind, wobei
in den Plattenzwischenräumen (17) ein fester Methanolsynthese-Katalysator angeordnet ist, welcher das Katalysatorbett CB1 (18a) bildet, und die erste Prozesseinheit (13) so konfiguriert ist, dass die Plattenzwischenräume (17) von dem Synthesegas-Strom (29) und von dem methanolhaltigen Produktstrom RP1 (30) von oben nach unten durchströmbar sind und die Platteninnenräume (17) von einem ersten Kühlmedien-Strom CM1 (33) von unten nach oben durchströmbar sind, wodurch der methanolhaltige Produktstrom RP1 (30) durch den ersten Kühlmedien-Strom CM1 (33) im Gegenstrom kühlbar ist, und wobei die erste Prozesseinheit (13) Mittel zum Abziehen des methanolhaltigen Produktstroms RP1 (30) aus der ersten Prozesseinheit (13) aufweist;
(c) eine unterhalb der ersten Prozesseinheit (13) angeordnete zweite Prozesseinheit (14a, 14b), wobei
die zweite Prozesseinheit (14a, 14b) als Plattenwärmeübertrager ausgestaltet ist, wobei die zweite Prozesseinheit (14a, 14b) dabei eine Vielzahl von vertikal und parallel zueinander angeordneten Platten (15) mit durchströmbaren Platteninnenräumen (16) aufweist, und zwischen benachbarten Platten (15) Plattenzwischenräume (17) ausgebildet sind, wobei
die zweite Prozesseinheit (14a, 14b) so konfiguriert ist, dass die Platteninnenräume (17) von einem zweiten Kühlmedien-Strom CM2 (28) von unten nach oben durchströmbar sind, und wobei
- (c1) die zweite Prozesseinheit (14a) als Kühlstufe ausgestaltet ist und dabei so konfiguriert ist, dass die Plattenzwischenräume (17) von dem methanolhaltigen Produktstrom RP1 (30) von oben nach unten durchströmbar sind, wodurch der methanolhaltige Produktstrom RP1 (30) durch den zweiten Kühlmedien-Strom CM2 (28) im Gegenstrom kühlbar ist, oder
- (c2) die zweite Prozesseinheit (14b) als Reaktorstufe RS2 ausgestaltet ist und dabei zur Synthese von Methanol aus dem aus der ersten Prozesseinheit (13) abziehbaren methanolhaltigen Produktstrom RP1 (30) an einem Katalysatorbett CB2 (18b) konfiguriert ist, wodurch ein methanolhaltiger Produktstrom RP2 (32) erzeugbar ist, und wobei
in den Plattenzwischenräumen (17) ein fester Methanolsynthese-Katalysator angeordnet ist, welcher das Katalysatorbett CB2 (18b) bildet, und die zweite Prozesseinheit (14b) so konfiguriert ist, dass die Plattenzwischenräume (17) von dem methanolhaltigen Produktstrom RP1 (30) und dem methanolhaltigen Produktstrom RP2 (32) von oben nach unten durchströmbar sind, wodurch der methanolhaltige Produktstrom RP2 (32) durch den zweiten Kühlmedien-Strom CM2 (28) im Gegenstrom kühlbar ist.

2. Methanolsynthese-Reaktor nach Anspruch 1, **dadurch gekennzeichnet, dass** die Platten der ersten Prozesseinheit (13) und/oder die Platten der zweiten Prozesseinheit (14a, 14b) als Kissenplatten ausgestaltet sind.

3. Methanolsynthese-Reaktor nach Anspruch 1 oder 2, **dadurch gekennzeichnet, dass** die zweite Prozesseinheit (14a, 14b) so konfiguriert ist, dass die Platteninnenräume (17) von dem Synthesegas-Strom (28) durchströmbar sind, wobei der Synthesegas-Strom (28) die Funktion des zweiten Kühlmedien-Stroms CM2 (28) erfüllt, wodurch ein vorgewärmter Synthesegas-Strom (29) erzeugbar ist.

4. Methanolsynthese-Reaktor nach Anspruch 3, **dadurch gekennzeichnet, dass** der Methanolsynthese-Reaktor Mittel zum Ausleiten des vorgewärmten Synthesegas-Stroms (29) aus der zweiten Prozesseinheit (14a, 14b) aufweist, sowie Mittel zum Einleiten des vorgewärmten Synthesegasstroms (29) in die erste Prozesseinheit (13) zum Erzeugen des methanolhaltigen Produktstroms RP1 (30) aufweist.

5. Methanolsynthese-Reaktor nach einem der vorhergehenden Ansprüche, **dadurch gekennzeichnet, dass** die Platteninnenräume (16) der ersten Prozesseinheit (13) und die Platteninnenräume (16) der zweiten Prozesseinheit (14a, 14b) zumindest teilweise fluchtend hintereinander angeordnet sind und/oder die Plattenzwischenräume (17) der ersten Prozesseinheit (13) und die Plattenzwischenräume (17) der zweiten Prozesseinheit (14a, 14b) zumindest teilweise fluchtend hintereinander angeordnet sind.

6. Methanolsynthese-Reaktor nach einem der vorhergehenden Ansprüche, **dadurch gekennzeichnet, dass** der Methanolsynthese-Reaktor eine Stützstruktur (19, 20) aufweist, wobei die Stützstruktur als zumindest teilweise unterhalb einer Prozesseinheit angeordnete Abstützung (19) und/oder als zumindest teilweise oberhalb einer Prozesseinheit angeordnete Aufhängung (20) ausgestaltet ist und dabei eine mechanische Verbindung zwischen einer Prozesseinheit (13, 14a, 14b) und dem Druckmantel (11) ausbildet.

7. Methanolsynthese-Reaktor nach Anspruch 6, **dadurch gekennzeichnet, dass** die erste Prozesseinheit (13) eine als zumindest teilweise unterhalb der ersten Prozesseinheit (13) angeordnete und als Abstützung (19) ausgebildete Stützstruktur aufweist und die zweite Prozesseinheit (14a, 14b) eine als zumindest teilweise oberhalb der zweiten Prozesseinheit (14a, 14b) angeordnete und als Aufhängung (20) ausgebildete Stützstruktur aufweist.

8. Methanolsynthese-Reaktor nach Anspruch 6, **dadurch gekennzeichnet, dass** die erste Prozesseinheit (13) eine als zumindest teilweise oberhalb der ersten Prozesseinheit (13) angeordnete und als Aufhängung ausgebildete Stützstruktur aufweist, und wobei die erste und die zweite Prozesseinheit über ein Verbindungselement (21) mechanisch miteinander verbunden sind.

9. Methanolsynthese-Reaktor nach einem der Ansprüche 3 bis 8, **dadurch gekennzeichnet, dass** der Methanolsynthese-Reaktor einen sich durch den Druckmantel (11) hindurch erstreckenden Synthesegaseintrittstutzen aufweist, wobei der Synthesegaseintrittstutzen fluidisch mit den Platteninnenräumen (16) der zweiten Prozesseinheit (14a, 14b) verbunden ist und zum Einleiten des Synthesegasstroms (28) in die zweite Prozesseinheit (14a, 14b) konfiguriert ist.

10. Methanolsynthese-Reaktor nach Anspruch 9, **dadurch gekennzeichnet, dass** die zweite Prozesseinheit (14a, 14b) in ihrem Sumpfbereich ein Verteilersystem (22) aufweist, welches fluidisch mit dem Synthesegaseintrittstutzen und den Platteninnenräumen (16) der zweiten Prozesseinheit (14a, 14b) verbunden ist und zum Verteilen des über den Synthesegaseintrittstutzen eintretenden Synthesegas-Stroms (28) auf die Platteninnenräume (16) der zweiten Prozesseinheit (14a, 14b) konfiguriert ist.

11. Methanolsynthese-Reaktor nach einem der Ansprüche 3 bis 10, **dadurch gekennzeichnet, dass** die zweite Prozesseinheit (14a, 14b) in ihrem Kopfbereich ein Sammlersystem (23) umfasst, welches fluidisch mit den Platteninnenräumen (16) der zweiten Prozesseinheit (14a, 14b) verbunden ist und zum Sammeln des aus den Platteninnenräumen (16) der zweiten Prozesseinheit (14a, 14b) austretenden, vorgewärmten Synthesegas-Stroms (29) konfiguriert ist.

12. Methanolsynthese-Reaktor nach Anspruch 11, **dadurch gekennzeichnet, dass** die erste Prozesseinheit (13) in ihrem Kopfbereich ein Verteilersystem (24) umfasst, welches fluidisch mit dem Sammlersystem (23) der zweiten Prozesseinheit (14a, 14b) verbunden ist und zum Verteilen des vorgewärmten Synthesegases (29) auf die Plattenzwischenräume (17) der ersten Prozesseinheit (13) konfiguriert ist.

13. Methanolsynthese-Reaktor nach Anspruch 12, **dadurch gekennzeichnet, dass** das Sammlersystem (23) der zweiten Prozesseinheit (14a, 14b) und das Verteilersystem (24) der ersten Prozesseinheit (13) über eine die erste Prozesseinheit umgehende und zumindest teilweise innerhalb des Druckmantels verlaufende Leitung verbunden sind.

14. Methanolsynthese-Reaktor nach einem der vorhergehenden Ansprüche, **dadurch gekennzeichnet, dass** der erste Kühlmedien-Strom CM1 (33) siedendes Kesselspeisewasser umfasst, der erste Kühlmedien-Strom CM1 (33) insbesondere beim Durchströmen der Platteninnenräume (17) der ersten Prozesseinheit (13) verdampfbar ist.

15. Methanolsynthese-Reaktor nach einem der vorhergehenden Ansprüche, **dadurch gekennzeichnet, dass** zwischen der ersten Prozesseinheit (13) und der zweiten Prozesseinheit (14a, 14b) innerhalb des Druckmantels (11) ein Zwischenraumbereich angeordnet ist, wobei der Zwischenraumbereich fluidisch mit den Plattenzwischenräumen (17) der ersten Prozesseinheit (13) und den Plattenzwischenräumen (17) der zweiten Prozesseinheit (14a, 14b) verbunden ist und zum Überführen des methanolhaltigen Produktstroms RP1 (30) von der ersten Prozesseinheit (13) in die zweite Prozesseinheit (14a, 14b) konfiguriert ist.

## Claims

1. Methanol synthesis reactor (1, 2, 3, 4) for producing methanol from a synthesis gas mixture comprising
(a) a pressure jacket (11) having an interior (12), wherein at least two fluidically interconnected process units are arranged one atop the other in the interior (12);
(b) a first process unit (13), wherein the first process unit is configured as reactor stage RS1 for synthesis of methanol from a synthesis gas stream (29) over a catalyst bed CB1 (18a), thus making it possible to produce a methanol-containing product stream RP1 (30), and
the first process unit (13) is in the form of a plate heat exchanger, wherein the first process unit (13) comprises a multiplicity of plates (15) arranged vertically and parallel to one another with traversable plate interiors (16) and plate interspaces (17) are present between adjacent plates, wherein
a solid methanol synthesis catalyst forming the catalyst bed CB1 (18a) is arranged in the plate interspaces (17) and the first process unit (13) is configured such that the plate interspaces (17) are traversable from top to bottom by the synthesis gas stream (29) and by the methanol-containing product stream RP1 (30) and the plate interiors (17) are traversable from bottom to top by a first cooling media stream CM1 (33), thus making the methanol-containing product stream RP1 (30) coolable in countercurrent by the first cooling media stream CM1 (33), and wherein
the first process unit (13) comprises means for withdrawing the methanol-containing product stream RP1 (30) from the first process unit (13);
(c) a second process unit (14a, 14b) arranged below the first process unit (13), wherein
the second process unit (14a, 14b) is in the form of a plate heat exchanger, wherein the second process unit (14a, 14b) comprises a multiplicity of plates (15) arranged vertically and parallel to one another with traversable plate interiors (16), and plate interspaces (17) are present between adjacent plates (15), wherein
the second process unit (14a, 14b) is configured such that the plate interiors (17) are traversable from bottom to top by a second cooling media stream CM2 (28) and wherein
- (c1) the second process unit (14a) is in the form of a cooling stage and configured such that the plate interspaces (17) are traversable from top to bottom by the methanol-containing product stream RP1 (30), thus making the methanol-containing product stream RP1 (30) coolable in countercurrent by the second cooling media stream CM2 (28) or
- (c2) the second process unit (14b) is in the form of reactor stage RS2 and configured for synthesis of methanol from the methanol-containing product stream RP1 (30) withdrawable from the first process unit (13) over a catalyst bed CB2 (18b), thus making it possible to produce a methanol-containing product stream RP2 (32), and wherein
a solid methanol synthesis catalyst forming the catalyst bed CB2 (18b) is arranged in the plate interspaces (17) and the second process unit (14b) is configured such that the plate interspaces (17) are traversable from top to bottom by the methanol-containing product stream RP1 (30) and the methanol-containing product stream RP2 (32), thus making the methanol-containing product stream RP2 (32) coolable in countercurrent by the second cooling media stream CM2 (28).

2. Methanol synthesis reactor according to Claim 1, **characterized in that** the plates of the first process unit (13) and/or the plates of the second process unit (14a, 14b) are in the form of pillow plates.

3. Methanol synthesis reactor according to Claim 1 or 2, **characterized in that** the second process unit (14a, 14b) is configured such that the plate interiors (17) are traversable by the synthesis gas stream (28), wherein the synthesis gas stream (28) fulfils the function of the second cooling media stream CM2 (28), thus making it possible to produce a preheated synthesis gas stream (29).

4. Methanol synthesis reactor according to Claim 3, **characterized in that** the methanol synthesis reactor comprises means for discharging the preheated synthesis gas stream (29) from the second process unit (14a, 14b) and comprises means for introducing the preheated synthesis gas stream (29) into the first process unit (13) to produce the methanol-containing product stream RP1 (30).

5. Methanol synthesis reactor according to any of the preceding claims, **characterized in that** the plate interiors (16) of the first process unit (13) and the plate interiors (16) of the second process unit (14a, 14b) are arranged at least partially in serial alignment and/or the plate interspaces (17) of the first process unit (13) and the plate interspaces (17) of the second process unit (14a, 14b) are arranged at least partially in serial alignment.

6. Methanol synthesis reactor according to any of the preceding claims, **characterized in that** the methanol synthesis reactor comprises a support structure (19, 20), wherein the support structure is in the form of a propping means (19) which is at least partially arranged below a process unit and/or in the form of a suspending means (20) arranged at least partially above a process unit and thus forms a mechanical connection between a process unit (13, 14a, 14b) and the pressure jacket (11).

7. Methanol synthesis reactor according to Claim 6, **characterized in that** the first process unit (13) has a support structure which is arranged at least partially below the first process unit (13) and is in the form of a propping means (19) and the second process unit (14a, 14b) has a support structure which is arranged at least partially above the second process unit (14a, 14b) and is in the form of a suspending means (20).

8. Methanol synthesis reactor according to Claim 6, **characterized in that** the first process unit (13) has a support structure which is arranged at least partially above the first process unit (13) and is in the form of a suspending means and wherein the first and the second process unit are mechanically connected to one another via a connecting element (21).

9. Methanol synthesis reactor according to any of Claims 3 to 8, **characterized in that** the methanol synthesis reactor comprises a synthesis gas inlet port extending through the pressure jacket (11), wherein the synthesis gas inlet port is fluidically connected to the plate interiors (16) of the second process unit (14a, 14b) and is configured for introducing the synthesis gas stream (28) into the second process unit (14a, 14b).

10. Methanol synthesis reactor according to Claim 9, **characterized in that** the second process unit (14a, 14b) comprises in its bottom region a distributor system (22) which is fluidically connected to the synthesis gas inlet port and the plate interiors (16) of the second process unit (14a, 14b) and is configured for distributing the synthesis gas stream (28) entering via the synthesis gas inlet port to the plate interiors (16) of the second process unit (14a, 14b).

11. Methanol synthesis reactor according to any of Claims 3 to 10, **characterized in that** the second process unit (14a, 14b) comprises in its top region a collector system (23) which is fluidically connected to the plate interiors (16) of the second process unit (14a, 14b) and is configured for collecting the preheated synthesis gas stream (29) exiting from the plate interiors (16) of the second process unit (14a, 14b).

12. Methanol synthesis reactor according to Claim 11, **characterized in that** the first process unit (13) comprises in its top region a distributor system (24) which is fluidically connected to the collector system (23) of the second process unit (14a, 14b) and configured for distributing the preheated synthesis gas (29) to the plate interspaces (17) of the first process unit (13).

13. Methanol synthesis reactor according to Claim 12, **characterized in that** the collector system (23) of the second process unit (14a, 14b) and the distributor system (24) of the first process unit (13) are connected via a conduit bypassing the first process unit and at least partially running inside the pressure jacket.

14. Methanol synthesis reactor according to any of the preceding claims, **characterized in that** the first cooling media stream CM1 (33) comprises boiling boiler feed water and the first cooling media stream CM1 (33) is especially evaporable upon traversal of the plate interiors (17) of the first process unit (13).

15. Methanol synthesis reactor according to any of the preceding claims, **characterized in that** an interspace region is arranged within the pressure jacket (11) between the first process unit (13) and the second process unit (14a, 14b), wherein the interspace region is fluidically connected to the plate interspaces (17) of the first process unit (13) and the plate interspaces (17) of the second process unit (14a, 14b) and is configured for transferring the methanol-containing product stream RP1 (30) from the first process unit (13) into the second process unit (14a, 14b).

## Revendications

1. Réacteur de synthèse de méthanol (1, 2, 3, 4) pour la production de méthanol à partir d'un mélange de gaz de synthèse, présentant
(a) une enveloppe de pression (11) avec un espace intérieur (12), au moins deux unités de traitement reliées fluidiquement entre elles étant agencées l'une au-dessus de l'autre dans l'espace intérieur (12) ;
(b) une première unité de traitement (13), la première unité de traitement étant configurée sous forme d'étage de réacteur RS1 pour la synthèse de méthanol à partir d'un courant de gaz de synthèse (29) sur un lit de catalyseur CB1 (18a), ce qui permet de produire un courant de produit contenant du méthanol RP1 (30), et
la première unité de traitement (13) étant conçue sous forme d'échangeur de chaleur à plaques, la première unité de traitement (13) présentant une pluralité de plaques (15) agencées verticalement et parallèlement les unes aux autres avec des espaces intérieurs de plaque (16) pouvant être traversés, et des espaces entre les plaques (17) étant réalisés entre des plaques voisines,
un catalyseur de synthèse de méthanol solide étant agencé dans les espaces entre les plaques (17), lequel forme le lit de catalyseur CB1 (18a), et la première unité de traitement (13) étant configurée de telle sorte que les espaces entre les plaques (17) peuvent être traversés de haut en bas par le courant de gaz de synthèse (29) et par le courant de produit contenant du méthanol RP1 (30), et les espaces intérieurs de plaque (17) peuvent être traversés de bas en haut par un premier courant de fluide de refroidissement CM1 (33), ce qui permet de refroidir le courant de produit contenant du méthanol RP1 (30) à contre-courant par le premier courant de fluide de refroidissement CM1 (33), et
la première unité de traitement (13) présente des moyens pour extraire le courant de produit contenant du méthanol RP1 (30) de la première unité de traitement (13) ;
(c) une deuxième unité de traitement (14a, 14b) agencée en dessous de la première unité de traitement (13),
la deuxième unité de traitement (14a, 14b) étant conçue sous forme d'échangeur de chaleur à plaques, la deuxième unité de traitement (14a, 14b) présentant une pluralité de plaques (15) agencées verticalement et parallèlement les unes aux autres avec des espaces intérieurs de plaque (16) pouvant être traversés, et des espaces entre les plaques (17) étant réalisés entre des plaques voisines (15),
la deuxième unité de traitement (14a, 14b) étant configurée de telle sorte que les espaces intérieurs de plaque (17) peuvent être traversés de bas en haut par un deuxième courant de fluide de refroidissement CM2 (28), et
- (c1) la deuxième unité de traitement (14a) étant conçue sous forme d'étage de refroidissement et étant configurée de telle sorte que les espaces entre les plaques (17) peuvent être traversés de haut en bas par le courant de produit contenant du méthanol RP1 (30), le courant de produit contenant du méthanol RP1 (30) pouvant ainsi être refroidi à contre-courant par le deuxième courant de fluide de refroidissement CM2 (28), ou bien
- (c2) la deuxième unité de traitement (14b) étant conçue sous forme d'étage de réacteur RS2 et étant configurée pour la synthèse de méthanol à partir du courant de produit contenant du méthanol RP1 (30) pouvant être extrait de la première unité de traitement (13) sur un lit de catalyseur CB2 (18b), ce qui permet de produire un courant de produit contenant du méthanol RP2 (32), et un catalyseur de synthèse de méthanol solide étant agencé dans les espaces entre les plaques (17), lequel forme le lit de catalyseur CB2 (18b), et la deuxième unité de traitement (14b) étant configurée de telle sorte que les espaces entre les plaques (17) peuvent être traversés de haut en bas par le courant de produit contenant du méthanol RP1 (30) et le courant de produit contenant du méthanol RP2 (32), le courant de produit contenant du méthanol RP2 (32) pouvant ainsi être refroidi à contre-courant par le deuxième courant de fluide de refroidissement CM2 (28).

2. Réacteur de synthèse de méthanol selon la revendication 1, **caractérisé en ce que** les plaques de la première unité de traitement (13) et/ou les plaques de la deuxième unité de traitement (14a, 14b) sont conçues sous forme de plaques à coussins.

3. Réacteur de synthèse de méthanol selon la revendication 1 ou 2, **caractérisé en ce que** la deuxième unité de traitement (14a, 14b) est configurée de telle sorte que les espaces intérieurs de plaque (17) peuvent être traversés par le courant de gaz de synthèse (28), le courant de gaz de synthèse (28) remplissant la fonction du deuxième courant de fluide de refroidissement CM2 (28), ce qui permet de produire un courant de gaz de synthèse préchauffé (29).

4. Réacteur de synthèse de méthanol selon la revendication 3, **caractérisé en ce que** le réacteur de synthèse de méthanol présente des moyens pour évacuer le courant de gaz de synthèse préchauffé (29) de la deuxième unité de traitement (14a, 14b), ainsi que des moyens pour introduire le courant de gaz de synthèse préchauffé (29) dans la première unité de traitement (13) pour produire le courant de produit contenant du méthanol RP1 (30).

5. Réacteur de synthèse de méthanol selon l'une quelconque des revendications précédentes, **caractérisé en ce que** les espaces intérieurs de plaque (16) de la première unité de traitement (13) et les espaces intérieurs de plaque (16) de la deuxième unité de traitement (14a, 14b) sont agencés au moins partiellement en alignement les uns derrière les autres et/ou les espaces entre les plaques (17) de la première unité de traitement (13) et les espaces entre les plaques (17) de la deuxième unité de traitement (14a, 14b) sont agencés au moins partiellement en alignement les uns derrière les autres.

6. Réacteur de synthèse de méthanol selon l'une quelconque des revendications précédentes, **caractérisé en ce que** le réacteur de synthèse de méthanol présente une structure de support (19, 20), la structure de support étant conçue sous forme de support (19) agencé au moins partiellement en dessous d'une unité de traitement et/ou sous forme de suspension (20) agencée au moins partiellement au-dessus d'une unité de traitement et formant ainsi une liaison mécanique entre une unité de traitement (13, 14a, 14b) et l'enveloppe de pression (11).

7. Réacteur de synthèse de méthanol selon la revendication 6, **caractérisé en ce que** la première unité de traitement (13) présente une structure de support agencée au moins partiellement au-dessous de la première unité de traitement (13) et réalisée sous forme de support (19) et la deuxième unité de traitement (14a, 14b) présente une structure de support agencée au moins partiellement au-dessus de la deuxième unité de traitement (14a, 14b) et réalisée sous forme de suspension (20).

8. Réacteur de synthèse de méthanol selon la revendication 6, **caractérisé en ce que** la première unité de traitement (13) présente une structure de support agencée au moins partiellement au-dessus de la première unité de traitement (13) et réalisée sous forme de suspension, et la première et la deuxième unité de traitement sont reliées mécaniquement entre elles par un élément de liaison (21).

9. Réacteur de synthèse de méthanol selon l'une quelconque des revendications 3 à 8, **caractérisé en ce que** le réacteur de synthèse de méthanol comprend une tubulure d'entrée de gaz de synthèse s'étendant à travers l'enveloppe de pression (11), la tubulure d'entrée de gaz de synthèse étant reliée fluidiquement aux espaces intérieurs de plaque (16) de la deuxième unité de traitement (14a, 14b) et étant configurée pour introduire le courant de gaz de synthèse (28) dans la deuxième unité de traitement (14a, 14b).

10. Réacteur de synthèse de méthanol selon la revendication 9, **caractérisé en ce que** la deuxième unité de traitement (14a, 14b) présente, dans sa zone de fond, un système de distribution (22) qui est relié fluidiquement à la tubulure d'entrée de gaz de synthèse et aux espaces intérieurs de plaque (16) de la deuxième unité de traitement (14a, 14b) et qui est configuré pour distribuer le courant de gaz de synthèse (28) entrant par la tubulure d'entrée de gaz de synthèse aux espaces intérieurs de plaque (16) de la deuxième unité de traitement (14a, 14b).

11. Réacteur de synthèse de méthanol selon l'une quelconque des revendications 3 à 10, **caractérisé en ce que** la deuxième unité de traitement (14a, 14b) comprend, dans sa zone de tête, un système de collecte (23) qui est relié fluidiquement aux espaces intérieurs de plaque (16) de la deuxième unité de traitement (14a, 14b) et qui est configuré pour collecter le courant de gaz de synthèse préchauffé (29) sortant des espaces intérieurs de plaque (16) de la deuxième unité de traitement (14a, 14b).

12. Réacteur de synthèse de méthanol selon la revendication 11, **caractérisé en ce que** la première unité de traitement (13) comprend, dans sa zone de tête, un système de distribution (24) qui est relié fluidiquement au système de collecte (23) de la deuxième unité de traitement (14a, 14b) et qui est configuré pour distribuer le gaz de synthèse préchauffé (29) aux espaces entre les plaques (17) de la première unité de traitement (13).

13. Réacteur de synthèse de méthanol selon la revendication 12, **caractérisé en ce que** le système de collecte (23) de la deuxième unité de traitement (14a, 14b) et le système de distribution (24) de la première unité de traitement (13) sont reliés par une conduite contournant la première unité de traitement et s'étendant au moins partiellement à l'intérieur de l'enveloppe de pression.

14. Réacteur de synthèse de méthanol selon l'une quelconque des revendications précédentes, **caractérisé en ce que** le premier courant de fluide de refroidissement CM1 (33) comprend de l'eau d'alimentation de chaudière en ébullition, le premier courant de fluide de refroidissement CM1 (33) pouvant notamment être vaporisé lors de son passage dans les espaces intérieurs de plaque (17) de la première unité de traitement (13).

15. Réacteur de synthèse de méthanol selon l'une quelconque des revendications précédentes, **caractérisé en ce qu'**une zone d'espace intermédiaire est agencée entre la première unité de traitement (13) et la deuxième unité de traitement (14a, 14b) à l'intérieur de l'enveloppe de pression (11), la zone d'espace intermédiaire étant reliée fluidiquement aux espaces entre les plaques (17) de la première unité de traitement (13) et aux espaces entre les plaques (17) de la deuxième unité de traitement (14a, 14b) et étant configurée pour transférer le courant de produit contenant du méthanol RP1 (30) de la première unité de traitement (13) à la deuxième unité de traitement (14a, 14b).
